# EUROPEAN PATENT APPLICATION

(11) **EP 4 549 459 A1**
(43) Date of publication of application: **07.05.2025**
(21) Application number: 23830253.3
(22) Date of filing: 27.06.2023
(51) Int. Cl.: C07K 16/00, C12N 15/62, C12N 15/86, C12N 15/861, A61K 48/00, A61K 39/395, A61P 9/10, A61K 47/64, A61P 27/02

(54) **AAV DRUG FOR TREATING ANGIOGENESIS-RELATED FUNDUS DISEASES**

(30) Priority: 27.06.2022 CN 202210733386
(71) Applicant: SHANGHAI REFRESHGENE THERAPEUTICS CO., LTD., Shanghai 201131 (CN)
(72) Inventor: TAN, Qingqiao, Shanghai 201131 (CN); CAO, Xi, Shanghai 201131 (CN); CHEN, Qiuyu, Shanghai 201131 (CN); ZHOU, Rui, Shanghai 201131 (CN)
(74) Representative: Bayramoglu et al.
(86) International application number: PCT/CN2023/102771
(87) International publication number: WO 2024/002076

(57) **Abstract**

The present invention belongs to the technical field of recombinant adeno-associated virus (rAAV) gene therapy, relates to the preparation of an rAAV-delivered Fc-engineered VEGF receptor fusion protein or an anti-VEGF antibody, and is applied to the treatment of angiogenesis-related fundus diseases, such as age-related macular degeneration, wet maculopathy, diabetic retinopathy and other diseases. Based on the aflibercept-expressing gene, the present invention uses recombinant AAV delivery to achieve long-term stable expression of a target gene in the RPE layer by means of genetic modification and vector optimization, thereby delivering an optimized target gene sequence to fundus cells of a patient.

## Description

### TECHNIACAL FIELD

The present invention belongs to the technical field of recombinant adeno-associated virus (rAAV) gene therapy, and relates to preparation of an rAAV-delivered Fc-engineered VEGF receptor fusion protein or anti-VEGF antibody, which is applied to treatment of neovascularization-related fundus diseases, such as treatment of age-related macular degeneration, wet macular degeneration, diabetic retinopathy and other diseases.

### BACKGROUND TECHNOLOGY

Neovascularization can occur in intraocular tissues such as the retina, choroid, macula, optic disc, cornea, iris and ciliary body, causing pathological changes such as tissue hemorrhage, exudation and hyperplasia in these parts, including age-related macular degeneration (AMD), diabetic retinopathy (DR), retinopathy of premature (ROP), etc., which seriously affects vision and is the main cause of blindness in the elderly.

AMD is divided into two main subtypes: non-neovascular (dry AMD) and neovascular (wet AMD). Vascular endothelial growth factor (VEGF) is one of the important proteins that promote angiogenesis. At present, anti-VEGF drug therapy (including Ranibizumab, Aflibercept, Bevacizumab, Conbercept and Brolucizumab, etc.) has become the standard treatment regimen for wet AMD treatment.

Diabetic retinopathy (DR) is a common microvascular complication of diabetes, with a high rate of blindness and a complex pathogenesis. The pathological features of DR are retinal neovascularization and blood-retinal barrier disruption. The application of anti-VEGF drug therapy is also relatively common.

In recent years, with the continuous progress of gene delivery systems and editing technology, the field of gene therapy has developed rapidly. At present, recombinant AAV (rAAV, recombinant adeno-associated virus) has become the main platform for in vivo gene therapy delivery. The rAAV-packaged genome has the AAV protein-coding sequence deleted and the therapeutic gene expression cassette added at the same time. The only virus-derived sequences are ITRs, which are essential for guiding genome replication and packaging in a vector production process. AAV has been widely used in basic research and clinical trials because of its advantages such as a wide host range, high safety, low immunogenicity, tissue tropism and possibility of long-term stable expression.

At present, anti-VEGF therapy has become the first-line treatment of neovascular ophthalmopathy. Drugs approved for clinical use include antibodies and antibody fragments (Bevacizumab, Ranibizumab and Brolucizumab), fusion proteins (Aflibercept and Conbercept) and nucleic acid aptamer Pegaptanib, and bispecific antibody Vabysmo (Faricimab-svoa).

Eylea (Aflibercept) is a recombinant fusion protein consisting of domain 2 of VEGFR-1 and domain 3 of VEGFR-2 fused with the Fc fragment of IgG1. It inhibits neovascularization and reduces vascular permeability by binding to vascular endothelial growth factor subtypes A and B (VEGF-A and VEGF-B) and placental growth factor (PlGF). The U.S. Food and Drug Administration (FDA) and the European Medicines Agency (EMA) successively approved intravitreal injection of Afliberceptin for the treatment of 3 indications, i.e. macular edema secondary to retinal vein occlusion, wet age-related macular degeneration and diabetic macular edema, before August 2014.

Conbercept (Langmu) is a type of Chinese biological product that has obtained the international common name of the World Health Organization. The drug is a type of genetically engineered antibody drug, which is similar in structure to Aflibercept, but the difference is that Conbercept includes the Ig-like region of the VEGF receptor. Such a structure can increase the affinity with VEGF, and may also block all subtypes of VEGF-A, VEGF-B and placental growth factor, increase the binding rate and prolong the half-life of the drug in the body, with a molecular weight of 142KD. This drug has been approved by China Food and Drug Administration for the treatment of wAMD since the end of 2013.

Although protein drug treatment methods such as Aflibercept are currently the standard treatment for neovascular ophthalmopathy, long-term anti-VEGF therapy may increase the incidence of RPE atrophy, choroidal atrophy and geographic atrophy. Multicenter clinical studies found that after seven years of anti-VEGF treatment, the vision of some patients dropped to the baseline or even below the baseline level, and was even accompanied by the occurrence of macular atrophy and fibrosis.

Moreover, standard treatment once every 4-8 weeks is actually difficult to maintain, and repeated injections can increase the risk of inflammation, infection and other side effects in some patients, with high treatment costs and poor patient acceptance.

Therefore, in order to improve the treatment effect for patients with neovascular ophthalmopathy, alternative or complementary treatments must be developed to reduce the number of administrations, even enable patients to benefit from one-time administration for life and improve patient acceptance.

### CONTENT OF THE INVENTION

In the present invention, based on the Aflibercept-expressing gene, long-term stable expression of a target gene in an RPE layer is realized by recombinant AAV delivery through gene modification and vector optimization, and the optimized target gene sequence is delivered to fundus cells of patients. "One-time administration, long-term effectiveness" is realized, patients are provided with a safer, more economical and more convenient excellent treatment approach, the burden of existing clinical treatment of neovascular ophthalmopathy is reduced, and unmet clinical needs are met.

Considering the good clinical performance of Aflibercept, as a candidate molecule for gene therapy delivery, it maintains efficacy with long-term expression in vivo while posing a low risk.

Choroidal neovascularization (CNV) is a hallmark lesion of exudative nAMD and an important cause of vision loss in the elderly. At present, laser-induced CNV animal models have been widely used for the efficacy evaluation of drugs for the treatment of AMD, including protein drugs such as Bevacizumab, Aflibercept and Ranibizumab. In the CNV models of nAMD induced by laser in rodents and non-human primates (NHP), the transient vascular leakage and neovascular response of the choroidal vasculature persist for 2-3 weeks (in rodents) or 6-8 weeks (in NHP) after the laser disruption of the Bruch's membrane, and then spontaneously regress.

Establishing a model of persistent and recurrent vascular leakage and neovascularization will greatly facilitate and accelerate the evaluation of long-acting interventions to address the multiple clinical manifestations of pathological vascular instability and neovascularization. A DL-α-aminoadipic acid (DL-AAA) model is a chronic leakage model that has been reported in rats and rabbits, and routine candidate drug screening has been performed in these species. DL-AAA is a selective glial cell toxin that has been reported to inhibit the action of glutamine synthetase and impair the broader retinal homeostasis function of Müller cells, resulting in glial dysfunction and death, thus leading to blood-retinal barrier breakdown. Two months after injection of D-LAAA, the subretinal blood-retinal barrier was disrupted in rats, and the vascular leakage and tortuosity were increased. Recently, several groups of studies have shown that 2-36 months after IVT administration of DL-AAA in rabbits, vascular leakage and RNV were increased, and anti-VEGF drugs such as Bevacizumab, Ranibizumab, Aflibercept and DARPins targeting VEGF-A165 inhibited such pathologies. The retinal vascular and neuronal anatomical structures of rats, rabbits and humans are different, but those between monkeys and humans are essentially the same. The retinal vasculature, retinal segmentation and basal layer boundaries, proportional abundance of retinal neurons and glial cell subtypes, as well as the presence of the macula are homologous in monkeys and humans. Meanwhile, preclinical models of chronic retinal vascular leakage and neovascularization allow for the efficacy screening of short-acting and long-acting anti-angiogenic compounds at multiple stages of disease development. The present invention will use an NHP model of chronic vascular leakage induced by DL-AAA for effect verification.

A first technical solution provided in the present invention is an Fc fragment mutant of human IgG1, obtained by undergoing at least one of mutations of:
T250A, L251A, M252L, 1253A/D/P, S254A, T256A, L309A, H310L/V/A/D/E/Q, Q311A, L314A, M428L/I, H433L/V/A, N434L/V/A, H435L/V/A, Y436A,
where a numbering is indicated by the EU index;
preferably, the mutant has one of single mutations of: H310A, H310L, H435A, H435L, I253A, I253D, I253P, H310D, H310E or H310Q;
more preferably, the mutant has one of single-point mutations of: H310A, H310E or H435A;
preferably, the mutant has one of double mutations of: M252L/M482L, M252L/M482I, M252L/M482L, T250A/H310L, L251A/H310L, I253A/H310L, S254A/H310L, T256A/H310L, L309A/H310L, H310L/Q311A, H310L/L314A, H310L/H433A, H310L/N434A, H310L/H435A, H310L/Y436A, I253A/H310A, I253A/H435A or H310A/H435A;
more preferably, the mutant has double mutations of: H310A/H435A;
preferably, the mutant has one of triple mutations of: M252L/H310L/M482L, M252L/H310V/ M482L, M252L/M482L/H433L, M252L/M482L/H433V, M252L/M482L/N434L or M252L/M482L/H435V; and
preferably, the mutant has one of quadruple mutations of: M252L/M482L/N434V/H435L, M252L/H310L/M482L/H433L, M252L/H310L/M482L/N434L or M252L/H310L/M482L/H435L.

More preferably, the Fc fragment mutant has a sequence shown in SEQ ID NO. 1, 2, 3 or 4.

A second technical solution provided in the present invention is a VEGF receptor recombinant fusion protein or an anti-VEGF recombinant antibody,
where the VEGF receptor recombinant fusion protein is formed by fusing domain 2 of VEGFR-1, domain 3 of VEGFR-2, and the Fc fragment mutant of IgG1 according to the first technical solution;
further, the domain 2 of VEGFR-1 is as shown in SEQ ID NO. 5;
further, the domain 3 of VEGFR-2 is as shown in SEQ ID NO. 6; and
preferably, the Fc fragment mutant of IgG1 is H310A (shown in SEQ ID NO. 1), H310E (shown in SEQ ID NO. 2), H435A (shown in SEQ ID NO. 3), H310A/H435A (shown in SEQ ID NO. 4).

More preferably, an amino acid sequence of the VEGF receptor recombinant fusion protein is as shown in SEQ ID NO. 7, 8, 9 or 10;
the anti-VEGF recombinant antibody is obtained by replacing an Fc fragment in an existing anti-VEGF antibody with the Fc fragment mutant of IgG1 according to the first technical solution;
further, the existing anti-VEGF antibody includes, but is not limited to, Bevacizumab, Ranibizumab and Brolucizumab; and
furthermore, the anti-VEGF recombinant antibody is obtained by replacing an Fc fragment of Bevacizumab with the Fc fragment mutant of IgG1 according to the first technical solution and, more preferably, has an amino acid sequence shown as SEQ ID NO. 11.

A third technical solution provided in the present invention is an AAV virus vector expression cassette for expressing the VEGF receptor recombinant fusion protein or the anti-VEGF recombinant antibody according to the second technical solution, including a structure represented by the following formula I from 5' to 3' ends:

ITR-E1-E2-E3-E4-ITR formula (I),

where
ITR is an inverted terminal repeat sequence;
E1 is a promoter;
E2 is a signal peptide;
E3 is a nucleotide sequence encoding the VEGF receptor recombinant fusion protein or the anti-VEGF recombinant antibody according to the second technical solution; and
E4 is a Poly A sequence.

Further, the ITR (inverted terminal repeat sequence) is from AAV1, AAV2, AAV3, AAV4, AAV5, AAV6, AAV7, AAV8 or AAV9, and preferably, the ITR sequence is selected from AAV2;
further, the promoter is a DNA sequence that can initiate transcription of a target gene, the sequence can be recognized by an RNA polymerase and start the transcription to synthesize RNA, and the promoter includes, but is not limited to, a natural, optimized or combined promoter;
furthermore, the promoter is preferably a CMV, CBA, EF1a, SV40, PGK1, Ubc, CAG, TEF1, U6 or H1 promoter;
further, a source of the signal peptide includes, but is not limited to, Human OSM, Gaussia luc or Albumin (HSA);
further, the Poly A sequence is selected from bGH PolyA, SV40 PolyA or hGH PolyA; and
further, the above expression cassette further includes a regulatory element, and the expression regulatory element includes, but is not limited to, a regulatory element with a function: (1) for regulating expression of a target protein, for example, IRES, for initiating translation of a downstream gene; (2) a regulatory element for expressing miRNA and siRNA sequences; (3) an intron; (4) a localization sequence for localizing and expressing a target protein into a nucleus, cytoplasm or various organelles, and secreting the target protein out of a cell; (5) the regulatory element can also be part of a kozak sequence, and the kozak sequence is G/N-C/N-C/N-ANNAUGG, for example, GCCACCAUGG, etc.; (6) an enhancer, which can be from an SV40 virus, a CMV virus or an adenovirus, etc.; and (7) the regulatory element can be a WPRE.

Further, the above expression cassette further includes a tag element, where the tag element includes, but is not limited to, FLAG, HA, MYC, a fluorescent protein, a luciferase, a SUMO protein, a ubiquitin protein, GST, etc.

Preferably, the AAV virus vector expression cassette for expressing the VEGF receptor recombinant fusion protein or the anti-VEGF recombinant antibody is used to express the VEGF receptor recombinant fusion protein or the anti-VEGF recombinant antibody according to the second technical solution on an AAV virus vector;
further, the AAV virus vector includes, but is not limited to, a pAAV-CMV, pX601, pX551, pAAV-MCS plasmid, etc.;
preferably, the AAV virus vector expression cassette for expressing the VEGF receptor recombinant fusion protein or the anti-VEGF recombinant antibody includes: ITR-CMV promoter-CMV enhancer-intron-signal peptide-Kozak sequence-VEGF receptor recombinant fusion protein/anti-VEGF recombinant antibody coding sequence-PolyA-ITR;
more preferably, the AAV virus vector expression cassette for expressing the VEGF receptor recombinant fusion protein has a sequence shown in SEQ ID NO. 12; and
more preferably, the AAV virus vector expression cassette for expressing the anti-VEGF recombinant antibody has a sequence shown in SEQ ID NO. 13.

A fourth technical solution provided in the present invention is an adeno-associated virus packaging vector system, including: the AAV virus vector expression cassette for expressing the VEGF receptor recombinant fusion protein or the anti-VEGF recombinant antibody according to the third technical solution, a vector carrying AAV rep and cap genes and a helper virus vector, where the above vector is packaged into an AAV virus;
further, the vector carrying AAV rep and cap genes includes, but is not limited to: AAV1, AAV2, AAV5, AAV8, AAV9, AAV-R100, AAV-NN, AAV-GL, AAV8-Y447F, AAV8-Y733F, AAV8-Y447F/Y733F, AAV-DJ or AAV7m8 vectors, etc.; and
further, the helper virus vector is an adenovirus or herpes virus helper virus vector, and preferably a pHelper plasmid.

A fifth technical solution provided in the present invention is a method for packaging an adeno-associated virus, where the adeno-associated virus packaging vector system according to the fourth technical solution is transferred into a host cell for virus packaging;
further, the AAV virus vector expression cassette for expressing the VEGF receptor recombinant fusion protein or the anti-VEGF recombinant antibody, the vector carrying the AAV rep and cap genes and the helper virus vector in the packaging vector system according to the fourth technical solution are transferred into a host cell for virus packaging;
further, the host cell is a cell line capable of viral replication and stable inheritance, including, but not limited to, cells such as Hela-S3, HEK-293, HEK-293T, HEK-293FT, Expi293F, A549 and Sf9; and
preferably, the host cell is an HEK-293 cell, an HEK-293T cell or an Expi293F cell.

A sixth technical solution provided in the present invention is an adeno-associated virus obtained by adopting the packaging method according to the fifth technical solution, where
preferably, the AAV virus vector expression cassette for expressing the VEGF receptor recombinant fusion protein shown in SEQ ID NO. 12, the AAV8 Rep-Cap plasmid and the pHelper plasmid are transferred into a host cell Expi293F cell for virus packaging; and
preferably, the AAV virus vector expression cassette for expressing the anti-VEGF recombinant antibody shown in SEQ ID NO. 13, the AAV8 Rep-Cap plasmid and the pHelper plasmid are transferred into a host cell Expi293F for viral packaging.

A seventh technical solution provided in the present invention is a preparation or formulation or medicament including the AAV virus vector expression cassette for expressing the VEGF receptor recombinant fusion protein or the anti-VEGF recombinant antibody according to the third technical solution, or the adeno-associated virus according to the sixth technical solution;
further, the preparation or formulation or medicament can be in any dosage form, including, but not limited to, an injection dosage form and an ointment dosage form; and
further, in the preparation or formulation or medicament, the above AAV virus vector expression cassette for expressing the VEGF receptor recombinant fusion protein or the anti-VEGF recombinant antibody, or the above adeno-associated virus is a sole active ingredient.

An eighth technical solution provided in the present invention is use of the AAV virus vector expression cassette for expressing the VEGF receptor recombinant fusion protein or the anti-VEGF recombinant antibody according to the third technical solution, or the adeno-associated virus according to the sixth technical solution in preparation of a preparation or formulation or medicament for treating a neovascularization-related fundus disease, particularly age-related macular degeneration, wet macular degeneration, diabetic retinopathy and other diseases, where
further, a method of administering the preparation or formulation or medicament is unilateral ocular administration or bilateral ocular administration;
furthermore, a route of administration is an injection route such as intravitreal injection, subretinal injection, suprachoroidal injection; and
furthermore, a total administration dosage for lifelong single or multiple administration is 1×10⁸-1×10¹³ viral genomes/eye.

### Beneficial effects:

1. in the present invention, through Fc mutation, the affinity with FcRn is reduced, the ocular PK behavior is improved, the entry into the blood is decreased, and the stability is not affected, the accumulation in the eye is significantly increased, and the half-life is prolonged. Even if the entry into the blood occurs, due to the reduced affinity with FcRn, the half-life in the circulatory system is shorter, thus avoiding causing other systemic safety risks;
2. through the combined design of Fc mutation and a vector element, continuous and stable expression in vivo is achieved with a lower dose of AAV virus, which has obvious advantages compared with the in vivo protein expression data of existing similar AAV products;
3. the combination of an rAAV vector element delivering an Fc mutation-engineered VEGF antagonist designed in the present invention has a more long-lasting drug efficacy compared with the control group of existing marketed protein drugs;
4. the AAV virus prepared in the present invention can provide an opportunity to relieve or cure neovascular fundus diseases for life by one-time administration, or reduce the number of administrations of protein drugs; and
5. the same combination design can adopt a variety of routes of administration such as intravitreal injection, subretinal injection and suprachoroidal injection.

### DESCRIPTION OF THE DRAWINGS

FIG. 1 shows results of a promoter optimization experiment,
   where each promoter and enhancer in FIG. 1 is represented as follows:
   - CMV: CMV enhancer + CMV promoter;
   - GNAT2: IRBP enhancer + GNAT2 promoter;
   - CBA: CMV enhancer + chicken-β actin promoter;
   - MPL: CMV enhancer + CMV promoter + TPL-MLP;
   - RPE65: CMV enhancer + RPE65 promoter.
FIG. 2 shows effects of different signal peptides on expression levels.
FIG. 3 shows statistics of quantitative results of supernatant expression of Fc mutant proteins.
FIG. 4 shows effects of different PolyA on expression results of ARPE-19 cells transiently transfected with different Aflibercept.
FIG. 5 shows effects of different PolyA on expression results of HEK293 cells transiently transfected with different Aflibercept.
FIG. 6 shows results of thermostability analysis of candidate Fc-engineered VEGF receptor recombinant fusion proteins at 37°C.
FIG. 7 shows results of thermostability analysis of candidate Fc-engineered VEGF receptor recombinant fusion proteins at 40°C.
FIG. 8 shows an inhibitory function of AAV vector expression products on HUVEC cell proliferation.
FIG. 9 shows protein expression levels at different time points after AAV injection in mice.
FIG. 10 shows FFA (fundus fluorescein angiography) maps (late stage).
FIG. 11 shows average scores of fundus light spot leakage in each group of animals.
FIG. 12 shows typical maps of fundus fluorescein angiography (FFA) 4 weeks after laser modeling.
FIG. 13 shows baseline changes of fluorescence leakage in a PRNV modeling and administration group.

### SPECIFIC IMPLEMENTATIONS

In order to make the purposes, technical solutions and advantages of the present application more clearly understood, the present application will be described in further detail below in conjunction with specific embodiments. It is to be understood that the specific embodiments described herein are merely for illustration of the present patent and are not intended to limit the present invention.

The present invention provides an optimized VEGF receptor recombinant fusion protein or anti-VEGF recombinant antibody delivered by AAV, delivering an optimized target gene sequence to an eye of a patient by means of subretinal, intravitreal or suprachoroidal injection, etc., so as to realize long-term stable expression of the target gene in the retina and enable a lifelong treatment with one low-dose administration. Patients are provided with safer, more convenient and more excellent treatment methods, and the burden of the current clinical treatment of neovascularization-related fundus diseases is relieved; and the situations of poor patient compliance and poor persistence are addressed, and the treatment outcome of patients is improved.

Fc fusion proteins refer to novel recombinant proteins produced by fusing a certain biologically active functional protein molecule with an Fc fragment of immunoglobulin (IgG, IgA, etc.) by genetic engineering and other technologies. They not only retain the biological activity of functional protein molecules, but also have some properties of antibodies, such as prolonging the half-life by binding to related Fc receptors and triggering antibody-dependent cell-mediated cytotoxic effects, etc., which are of great significance in the diagnosis and treatment of diseases. FcRn is an IgG antibody receptor located on the surface of the cell membrane. Its protein structure is similar to that of the MHC-I molecule, and it is mainly expressed in endothelial cells (it can also be detected in other tissues or cells). Its structure includes a heterodimer composed of an α chain and β2 microglobulin. FcRn can bind to the Fc part of IgG, preventing IgG molecules from being cleaved by lysosomes, which can play a role in increasing the half-life of IgG in vivo, and participate in the metabolic process of transportation, maintenance and distribution of IgG in vivo. The Fc part of IgG interacts with the neonatal Fc receptor (FcRn, Brambell receptor), preventing its degradation in lysosomes and enabling the long-term persistence of antibodies in serum. Other biological effects of FcRn include perinatal IgG transfer, antibody-mediated antigen presentation, and IgG transfer across epithelial and endothelial barriers. Altering the IgG: FcRn interaction has been increasingly investigated for improving the therapeutic or diagnostic applications of antibodies. The in vivo action pathway of intraocular injection of IgG or Fc fusion proteins is mainly mediated by FcRn on retinal RPE and endothelial cells to cross the blood-retinal barrier and is gradually eliminated (elimination) after entering the systemic circulation. Therefore, FcRn mediates the blood entry and elimination of IgG or Fc fusion proteins in ocular metabolism.

The optimized VEGF receptor recombinant fusion protein according to the present invention includes the recombinant protein of human vascular endothelial growth factor (VEGF) receptor 1 Domain 2 and receptor 2 Domain 3 with the human immunoglobulin Fc segment mutant. In one embodiment of the present invention, multiple groups of Fc single-point or multi-point mutants are designed, and the selection of mutation sites in this invention not only takes into account the FcRn affinity, but preferably also takes into account the stability and persistence of the expression of the target protein by the rAAV composition in vitro and in vivo. Specific mutation sites of the Fc mutant include: T250A, L251A, M252L, I253A/D/P, S254A, T256A, L309A, H310L/V/A/D/E/Q, Q311A, L314A, M428L/I, H433L/V/A, N434L/V/A, H435L/V/A, Y436A, etc. In one embodiment of the present invention, preferred mutation sites are single-point mutations, including H310A, H310L, H435A, H435L, I253A, I253D, I253P, H310D, H310E, H310Q. In another embodiment of the present invention, preferred mutation sites are double-point mutations, including M252L and M482L, M252L and M482I, M252L and M482L, T250A and H310L, L251A and H310L, I253A and H310L, S254A and H310L, T256A and H310L, L309A and H310L, H310L and Q311A, H310L and L314A, H310L and H433A, H310L and N434A, H310L and H435A, H310L and Y436A, I253A and H310A, I253A and H435A, H310A and H435A, etc. In another embodiment of the present invention, preferred mutation sites are triple-point mutations, including M252L, H310L and M482L, M252L, H310V and M482L, M252L, M482L and H433L, M252L, M482L and H433V, M252L, M482L and N434L, M252L, M482L and H435V. In another embodiment of the present invention, preferred mutation sites are quadruple-point mutations, including M252L, M482L, N434V and H435L, M252L, H310L, M482L and H433L, M252L, H310L, M482L and N434L, M252L, H310L, M482L and H435L. In another embodiment of the present invention, more preferred single-point mutation sites are H310A, H310E, H435A, and the obtained Fc mutant has an amino acid sequence shown in SEQ ID NO. 1, 2 or 3. In another embodiment of the present invention, a more preferred double-point mutation site is H310A/H435A, and the Fc mutant has an amino acid sequence shown in SEQ ID NO. 4.

The above Fc mutant is of a mutation that occurs based on the following wild-type Fc fragment of human IgG1 (SEQ ID NO. 15):

DGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPG.

In the present invention, the numbering of amino acid residues in the Fc fragment is the numbering of the heavy chain of immunoglobulin according to the EU index in Kabat et al., Sequences of Proteins of Immunological Interest, 5th ed. Public Health Service, National Institutes of Health, Bethesda, Maryland (1991) (expressly incorporated herein by reference). "As per the EU indexed/numbering" herein refers to the EU coding sequence number of residues in the constant region of a human IgG1 antibody". This numbering is well known to those skilled in the art and is frequently used in the art. It can also be referred to at the following website: https://www.imgt.org/IMGTScientificChart/Numbering/Hu_IGHGnber.html.

More preferably, in another embodiment of the present invention, an amino acid sequence of the VEGF receptor recombinant fusion protein is as shown in SEQ ID NO. 7-10.

The anti-VEGF recombinant antibody according to the present invention is obtained by replacing an Fc fragment in an existing anti-VEGF antibody with the aforementioned Fc fragment mutant of IgG1; in another embodiment of the present invention, the existing anti-VEGF antibody includes, but is not limited to, Bevacizumab, Ranibizumab and Brolucizumab; and in another embodiment of the present invention, the anti-VEGF recombinant antibody is obtianed by replacing an Fc fragment of Bevacizumab with an Fc fragment mutant H310E of IgG1 according to the first technical solution, and preferably, it has an amino acid sequence shown in SEQ ID NO. 11.

The present invention further provides a vector expression cassette, including a structure represented by formula I from 5' to 3' ends:

ITR-E1-E2-E3-E4-ITR (I),

where:
ITR is an inverted terminal repeat sequence; E1 is a promoter (including a natural, optimized or combined promoter); E2 is a signal peptide; E3 is a nucleotide sequence encoding the VEGF receptor recombinant fusion protein or the anti-VEGF recombinant antibody according to the present invention; and E4 is a Poly A sequence.

In some implementations, the ITR sequence (inverted terminal repeat sequence) is from AAV1, AAV2, AAV3, AAV4, AAV5, AAV6, AAV7, AAV8 or AAV9; and preferably, it is from AAV2; and
in some implementations, the nucleic acid sequence has a promoter. The promoter is used to initiate the expression of the nucleic acid sequence to which it is linked, the promoter can be constitutive or inducible, and the promoter can be a promoter that is expressed specifically in retinal pigment epithelial cells or can also be a ubiquitous promoter. Preferred promoters include various eukaryotic promoters, for example, CMV, EF1a, CBA, SV40, PGK1, Ubc, CAG, TEF1, U6, H1, etc. Most recombinant AAV vector promoters use a broad-spectrum CMV (CMV enhancer and CMV promoter), CBA (CMV enhancer and chicken β9-actin promoter), PGK promoter and EF1a promoter. More preferably, the promoter is a CMV promoter.

The signal peptide is a short peptide chain (5-30 amino acids in length) that directs the transfer of the newly synthesized protein to the secretory pathway. It generally refers to an amino acid sequence at the N-terminus (sometimes not necessarily at the N-terminus) of the newly synthesized polypeptide chain used to direct the transmembrane transfer (localization) of proteins. After the start codon, there is an RNA region encoding a hydrophobic amino acid sequence. The amino acid sequence is called the signal peptide sequence, which is responsible for guiding proteins into suborganelles containing different membrane structures in cells. The signal peptide includes three regions: a positively charged N-terminus, called the basic amino terminus; an intermediate hydrophobic sequence that is mainly composed of neutral amino acids and can form an α-helix structure, which is the main functional region of the signal peptide; and a long negatively charged C-terminus that contains small-molecule amino acids and is the cleavage site of the signal sequence. The C-terminus is also called a processing region. When the signal peptide sequence is synthesized, it is recognized by the signal recognition particle (SRP), and protein synthesis is paused or slowed down. The signal recognition particle carries the ribosome to the endoplasmic reticulum, and protein synthesis resumes. Under the guidance of the signal peptide, the newly synthesized protein enters the endoplasmic reticulum lumen. The signal peptide sequence is cleaved off under the action of the signal peptidease. If the stop-transfer sequence exists at the C-terminus of the nascent peptide chain, the signal peptide sequence may not be cleaved off by the signal peptidease.

In some embodiments of the present invention, commonly used eukaryotic expression signal peptides (see Table 1) are selected, and the SP1-SP13 signal peptides are inserted into the N-terminus of the target gene by the PCR method and homologously recombined into the PTT5 vector double-digested by EcoRI and HindIII. After transformation into *Escherichia coli,* sequencing is performed to verify the correctness of the sequence. Then a large number of plasmids are extracted with endotoxin removal, and the HEK293E cells are transiently transfected by the PEI method. The cells are cultured in CD05 medium containing glutamine glutamine for 5 days, and then the cell supernatant is taken to detect the protein expression level. Meanwhile, the target protein in the cell culture supernatant is purified using Protein A affinity chromatography. The quantification of the protein is performed by the bicinchoninic acid (BCA) method. A preferred signal peptide sequence is obtained according to the expression level of the target protein.

In another embodiment, a preferred signal peptide is from Human OSM, Gaussia luc or Albumin (HSA). In another embodiment, a more preferred signal peptide is from Gaussia luc.

Polyadenylation refers to covalent linkage of polyadenylic acid to messenger RNA (mRNA) molecules. In the process of protein biosynthesis, it is part of the way to generate mature mRNA ready for translation. In eukaryotes, polyadenylation is a mechanism that causes mRNA molecules to be interrupted at their 3' end. The polyadenylate tail (or poly A tail) protects mRNA from exonuclease attacks and is very important for transcription termination, export of mRNA from the nucleus and translation.

In certain embodiments, main choices for PolyA sequences are bGH PolyA (derived from pCMV3) and SV40 PolyA (derived from pCGS3) sequences. Different PolyA sequences may increase transcription levels through their interaction with other expression elements. In one embodiment of the present invention, three different PolyA sequences, i.e. bGH PolyA, SV40 PolyA and hGH PolyA, are tested to obtain optimal gene expression and virus production. In another embodiment of the present invention, preferred PolyA sequences are bGH PolyA and hGH PolyA. In another embodiment of the present invention, a preferred PolyA sequence is bGH PolyA.

In certain embodiments, the above expression cassette further includes a regulatory element, and the expression regulatory element includes, but is not limited to, a regulatory element with a function: (1) for regulating expression of a target protein, for example, IRES, for initiating translation of a downstream gene; (2) a regulatory element for expressing miRNA and siRNA sequences; (3) an intron, also known as an intervening sequence, refers to a fragment of a gene or mRNA molecule that has no coding effect; (4) a localization sequence for localizing and expressing a target protein into a nucleus, cytoplasm or various organelles, and secreting the target protein out of a cell; (5) the regulatory element can also be part of a Kozak sequence, and the Kozak sequence is a nucleic acid sequence located behind the 5' end cap structure of eukaryotic mRNA, usually GCCACCAUGG. It can bind to translation initiation factors to mediate the translation initiation of mRNA containing the 5' cap structure, corresponding to the SD sequence of prokaryotes, and exists in a sequence of eukaryotic mRNA, which plays an important role in the initiation of translation. The Kozak sequence is G/N-C/N-C/N-ANNAUGG, e.g. GCCACCAUGG; (6) an enhancer, which can be from an SV40 virus, a CMV virus or an adenovirus, etc.; and (7) the regulatory element can be a WPRE.

In certain embodiments, the above expression cassette further includes a tag element, where the tag element includes, but is not limited to, e.g. FLAG, HA, MYC, a fluorescent protein, a luciferase, a SUMO protein, a ubiquitin protein, GST, etc.

In one implementation, the present invention discloses a recombinant viral vector including the following elements: (a) a first AAV2 inverted terminal repeat (ITR) sequence, (b) a CMV enhancer and promoter, (c) a chimeric intron, (d) a Kozak sequence, (e) a signal peptide sequence, (f) a VEGF receptor recombinant fusion protein/anti-VEGF recombinant antibody coding sequence, (g) a WPRE sequence, (h) a bGH polyA sequence, and (i) a second AAV2 ITR.

In one implementation, the above optimized VEGF receptor recombinant fusion protein/anti-VEGF recombinant antibody is expressed on an AAV viral vector including, but not limited to, pAAV-CMV, pAAV-MCS plasmids; and preferably, the AAV viral vector is pAAV-CMV.

The present invention further provides an AAV virus packaged by the above vector expressing the optimized VEGF receptor fusion protein/anti-VEGF recombinant antibody. The AAV virus can be prepared using a standard method disclosed in the art. Reference can be made to "AAV Production Everywhere: A Simple, Fast, and Reliable Protocol for In-house AAV Vector Production Based on Chloroform Extraction".

A replication-defective recombinant AAV can be prepared by co-transfecting three plasmids into a cell line infected with a human helper virus (e.g. adenovirus): a plasmid containing a nucleic acid sequence of interest flanked by two AAV inverted terminal repeat (ITR) regions, a helper packaging plasmid, and a plasmid carrying the AAV capsidization genes (rep and cap genes). Then the resulting AAV recombinant virus is purified by a standard technique. Preferably, the recombinant adeno-associated virus is a single-stranded AAV.

In some embodiments, a coding gene fragment of the optimized VEGF receptor recombinant fusion protein/anti-VEGF recombinant antibody is packaged into a viral particle (e.g., an AAV serotype viral particle including, but not limited to, AAV1, AAV2, AAV3, AAV4, AAV5, AAV6, AAV7, AAV8, AAV9, AAV10, AAV11, AAV12, AAV13, AAV14, AAV15, AAV16, AAV7m8, AAV PHP.eB, etc.). Therefore, the present disclosure includes a recombinant viral particle containing any of the vectors described herein. Preferably, the recombinant adeno-associated virus serotypes are AAV2 and AAV8. More preferably, the recombinant adeno-associated virus serotype is AAV8.

A preferred viral vector for delivering a nucleic acid sequence encoding the VEGF receptor recombinant fusion protein/anti-VEGF recombinant antibody is an AAV vector, such as self-complementary AAV (scAAV), single-stranded AAV (ssAAV), pAAV-CMV, pAAV-MCS, etc.

Selective targeting can be achieved using a specific AAV serotype (AAV1, AAV2, AAV3, AAV4, AAV5, AAV6, AAV7, AAV8, AAV9, AAV10, AAV11, AAV12, AAV13, AAV14, AAV15, AAV16, AAV7m8, AAV PHP.eB) or any one of other modified serotypes.

The host cell used for assembly of the above virus in the present invention is used for AAV vector-transduced cells to express the Fc-engineered VEGF receptor recombinant fusion protein and anti-VEGF antibody. Preferably, the host cell is a mammalian cell (preferably a cell derived from the human body, more preferably a human optic nerve cell or a photoreceptor cell), which increases the expression level of the VEGF receptor fusion protein and the anti-VEGF antibody, and embodies the specific expression of the AAV vector molecule of the present invention. The host cell includes, but is not limited to, a cell such as Hela-S3, HEK-293, HEK-293T, HEK-293FT, Expi293F, A549 and Sf9; and in some implementations of the present invention, a preferred host cell is HEK-293, an HEK-293T cell or an Expi293F cell.

In one implementation, the present invention discloses the following recombinant adeno-associated virus: AAV8-Afli Fc Variant10. The recombinant virus is an AAV8 serotype virus, the vector carrying the transgene has a sequence set forth in SEQ ID NO: 12, the transgene carried is a coding gene for the VEGF receptor recombinant fusion protein, and the H310E mutation modification is performed at the 310th position in the Fc region of the Aflibercept fusion protein gene.

In one implementation, the present invention discloses the following recombinant virus: AAV8-Bevacizumab Fc Variant10. The recombinant virus is an AAV8 serotype virus, the vector carrying the transgene has a sequence set forth in SEQ ID NO: 13, the transgene carried is a mutant gene of a Bevacizumab monoclonal antibody, and the H310E mutation modification is performed at the 310th position in the Fc region of the Bevacizumab gene.

The recombinant virus constructed in the present invention is suitable for use in vivo or ex vivo, preferably for one-dose administration in the vitreous body, subretinal space or suprachoroidal space of patients with neovascularization-related fundus diseases (including age-related macular degeneration, wet macular degeneration, diabetic macular edema). Preferably, a safe and effective dose range for administration to mice is 1×10⁶-1×10¹¹ viral genomic copies/eye, a safe and effective dose range for administration to NHP is 1×10⁸-1×10¹³ viral genomic copies/eye, and a safe and effective dose range for administration to humans is 1×10⁸-1×10¹³ viral genomic copies/eye (genomic copies, GC for short).

In one implementation, the present invention provides a composition including a polynucleotide or viral vector or adeno-associated virus of the present invention.

In one implementation, the present invention provides a method for preparing a composition including a polynucleotide or viral vector or adeno-associated virus of the present invention.

In one implementation, the present invention provides a route of administration for a composition including a polynucleotide or viral vector or adeno-associated virus of the present invention via the subretinal space.

In one implementation, the present invention provides a route of administration for a composition including a polynucleotide or viral vector or adeno-associated virus of the present invention by intravitreal injection.

In one implementation, the present invention provides a route of administration for a composition including a polynucleotide or viral vector or adeno-associated virus of the present invention via the suprachoroidal space.

In one implementation, the present invention provides a method for observing after administration to mice/NHP/humans, including optical coherence tomography (OCT) and fundus fluorescein angiography (FFA).

In one implementation, the present invention provides a method for detecting expression of Aflibercept protein in the eyeball tissue after administration to mice.

### Formulations and compositions

The present invention provides a formulation or composition containing the adeno-associated virus vector or virus according to the present invention, and a pharmaceutically acceptable carrier or excipient.

In another preferred embodiment, the pharmaceutical formulation is used for treating an ocular disease, and preferably the pharmaceutical formulation is used for treating a VEGF-related ocular disease, preferably used for treating macular degeneration and/or diabetic retinopathy, and more preferably used for treating wet age-related macular degeneration or wet macular degeneration.

For the convenience of clinical application, the pharmaceutical composition of the present invention can be filled in a sealed vial or a pre-filled syringe. The vial or pre-filled syringe can be packaged in a medicine packaging box to facilitate storage and use. The pharmaceutical preparation and composition of the present invention are stored in an ultra-low temperature refrigerator below -60°C and transported by adopting a dry-ice cold chain.

The dosage form of the pharmaceutical formulation or formulation composition can be a liquid or solid, such as a powder, gel or paste. Preferably, the composition is a liquid, and more preferably an injection.

The AAV vector formulation provided in the present invention has a titer in a range of 1×10¹⁰-1×10¹⁴ GC/ml, and preferably 1×10¹²-1×10¹³ GC/ml.

The formulation includes a pharmaceutically acceptable excipient. **In** some cases, the excipient includes a surfactant or a stabilizer. In the present invention, the surfactant is selected from
a polysorbate, sodium dodecyl sulfate, sodium lauryl sulfate, lauryl dimethylamine oxide, a polyethoxylated alcohol, polyoxyethylene sorbitol, octylphenol polyether, Brij, pluronic and polyoxyl castor oil. In some cases, a pharmaceutically acceptable excipient includes phenol, mannitol, sorbitol, sucrose or sodium chloride.

The "active ingredient" in the pharmaceutical composition according to the present invention refers to the vector or AAV virus according to the present invention. The "active ingredient", preparation and/or composition according to the present invention can be used for treating an ocular disease. A "safe and effective amount" refers to an amount of the active ingredient sufficient to significantly ameliorate the condition or symptom without causing serious side effects. A "pharmaceutically acceptable carrier or excipient" refers to one or more compatible solid or liquid filler or gel substances which are suitable for human use and must have sufficient purity and sufficiently low toxicity. "Compatibility" herein is meant that components of the composition can be admixed with the active ingredient of the present invention and with each other without significantly reducing the efficacy of the active ingredient.

### Treatment methods

The present invention provides a method for treating VEGF-mediated neovascular ophthalmopathy, including introducing a virus or viral vector including a nucleic acid sequence encoding an Fc-engineered VEGF receptor recombinant fusion protein or anti-VEGF recombinant antibody into an eye. The method may include injecting a nucleic acid vector into the subretinal, intravitreal or suprachoroidal space, and the nucleic acid vector targets an RPE cell or a photoreceptor cell.

The present invention provides a nucleic acid vector for use in a method for treating neovascular ophthalmopathy by delivering a nucleic acid sequence encoding a therapeutic protein to a retinal cell, including a nucleic acid sequence encoding an Fc-engineered VEGF receptor recombinant fusion protein or anti-VEGF recombinant antibody. The composition of the present invention can be administered alone or in combination with other therapeutic drugs (e.g., formulated in the same pharmaceutical composition).

As described in the present invention, treating a disease means administering the nucleic acid or vector or virus as described herein to ameliorate or alleviate one or more symptoms of the disease, including reducing vascular leakage, reducing neovascularization, etc.

Preferably, the method of the present invention includes introducing the nucleic acid sequence encoding the Fc-engineered VEGF receptor recombinant fusion protein or anti-VEGF recombinant antibody into the subretinal space of the eye.

Preferably, the method of the present invention includes contacting a cell with a vector (preferably a virus, more preferably an adeno-associated virus) including the nucleic acid sequence encoding the Fc-engineered VEGF receptor recombinant fusion protein or anti-VEGF recombinant antibody. Preferably, the cell is a retinal cell, preferably a pigment epithelial cell, a cone cell, a rod cell, a bipolar cell, a horizontal cell, a ganglion cell and/or an amacrine cell.

The recombinant adeno-associated virus vector of the present invention can effectively inhibit the expression of VEGF in animal eyes, reduce leakage and neovascularization caused by laser injury, and also reduce retinal vasculopathy caused by DL-AAA, so it can be used in preparation of a medicament for treating a VEGF-related disease, and treating a human VEGF-related ocular disease, and has broad market prospects.

When the viral vector including an effective amount of the nucleic acid sequence provided in the present invention is used for treating a human VEGF-related ocular disease, a clinical administration dose is 1×10⁸-1×10¹³ GC of the virus, an ocular injection volume is 50-300 µl, and a route of administration is subretinal, intravitreal or suprachoroidal injection.

The present invention will be further described below in conjunction with specific examples. The examples described are part of the examples of the present invention, rather than all of the examples. It is to be understood that the following examples are set forth to provide a complete disclosure and description to those of ordinary skill in the art to which the present invention belongs on how to utilize the method and composition of the present invention, and are not intended to limit the scope of the present invention. Based on the examples in the present invention, all other examples obtained by those of ordinary skill in the art without making creative efforts belong to the scope of protection of the present invention.

The mutant names and mutant information involved in the present invention and examples are as shown in the following table:

**Mutant comparison table**

| Mutant name | Fc mutation site | SEQ ID NO. |
|---|---|---|
| Variant1 | H310A | 7 |
| Variant2 | H435A | 9 |
| Variant3 | I253A | |
| Variant4 | I253A, H310A | |
| Variant5 | I253A, H435A | |
| Variant6 | H310A, H435A | 10 |
| Variant7 | I253D | |
| Variant8 | I253P | |
| Variant9 | H310D | |
| Variant10 | H310E | 8 |
| Variant11 | H310Q | |
| Variant12 | M428L, N434S | |

| | | |
|---|---|---|
| Note: the mutants Variant1-12 in the table are Aflibercept mutants obtained by performing corresponding mutations to the Fc fragment of Eylea (Aflibercept) shown in SEQ ID NO. 14. | | |

### Example 1 Promoter screening of gene expression cassettes

Most recombinant AAV vector promoters use a broad-spectrum CMV (CMV enhancer and CMV promoter), CBA (CMV enhancer and chicken-β actin promoter). It has been reported in the literature that optimized retinal cell-specific promoters may more efficiently express target proteins in specific tissues. In the experiment, we used pAAV-MCS plasmids as the basis and replaced promoter elements. CBA, CMV + TPL-MLP (TPL-MLP sequence derived from pTT5 vector), RPE65 (GenBank: AF304008.1) and IRBPe/GNAT2 (GenBank: X53044.1, GenBank: U66698.1) promoters were selected for the study. The amino acid sequence of Aflibercept was obtained from the DrugBank database (accession number DB08885) and was codon-optimized according to the codon usage preference of *Homo sapiens,* and the sequence was cloned into the pTT5 expression vector.

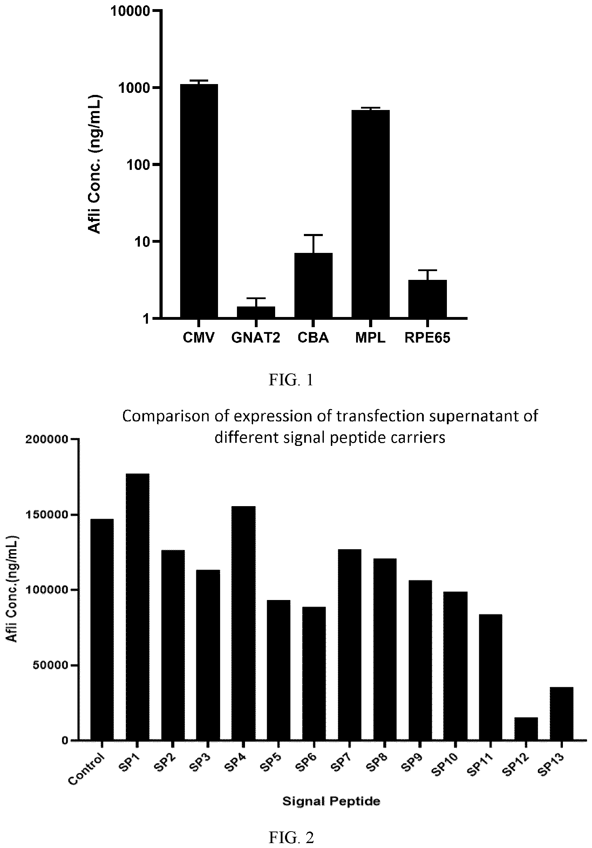

The specific experimental steps were as follows: 1) the Aflibercept sequence was optimized and synthesized, the RPE65 and IRBPe/GNAT2 promoter sequences were synthesized; 2) the RPE65 and IRBPe/GNAT2 promoter sequences were amplified by the PCR method, and homologous recombination with the pAAV-MCS vector digested by NcoI and SacI, MluI and SacI was performed sequentially. After transformation into *Escherichia coli,* sequencing and restriction enzyme digestion verification were performed to obtain pAAV-RPE65-MCS and pAAV-IRBPe/GNAT2-MCS vectors. 3) pAAV-RPE65-MCS, pAAV-IRBPe/GNAT2-MCS and pAAV-MCS were double-digested with EcoRI and HindIII. After the target gene sequence of Aflibercept was amplified by the PCR method, the Aflibercept sequence was inserted into the above digested vectors by a homologous recombination method. After transformation into *Escherichia coli,* sequencing and restriction enzyme digestion verification were performed to obtain pRPE65-AA V-Afli YB, pGNAT2-AAV-Afli and pAAV-Afli YB vectors. 4) CBA and Aflibercept sequences were amplified by the PCR method. After overlap PCR, the inserted sequences were homologously recombined into the pAAV-MCS vector digested by NcoI and HindIII. After transformation into *Escherichia coli,* sequencing and restriction enzyme digestion verification were performed to obtain the pCBA-AAV-Afli YB vector. 5) The sequences of CMV + TPL/MLP and Aflibercept were amplified by the PCR method. After overlap PCR, the inserted sequences were homologously recombined into the pAAV-MCS vector. After transformation into *Escherichia coli,* sequencing and restriction enzyme digestion verification were performed to obtain the pMPL-AAV-Afli YB vector. 6) A large number of plasmids obtained in steps 3, 4 and 5 were extracted with endotoxin removal and then transiently transfected into ARPE-19 cells. After 72 hours, the expression level of Aflibercept in the cell supernatant was detected by ELISA. The experimental results are shown in FIG. 1 showing results of a promoter optimization experiment. The results indicated that the protein expression level of the sequence containing the CMV promoter was significantly higher than that of other promoter sequences.

### Example 2 Screening of signal peptide sequences

Commonly used eukaryotic expression signal peptides (see Table 1) were selected, and the SP1-SP13 signal peptides were inserted into the N-terminus of the Aflibercept target gene shown in SEQ ID NO. 14 by the PCR method and homologously recombined into the PTT5 vector double-digested by EcoRI and HindIII. After transformation into *Escherichia coli,* sequencing was performed to verify the correctness of the sequence. Then a large number of plasmids were extracted with endotoxin removal, and the HEK293E cells were transiently transfected by the PEI method. The cells were cultured in CD05 medium containing glutamine for 5 days, and then the cell supernatant was taken and the expression level of Aflibercept was detected by ELISA. Meanwhile, the target protein in the cell culture supernatant was purified using Protein A affinity chromatography. The quantification of the protein was detected by the BCA method (Pierce^{™} Rapid Gold BCA protein assay kit). A preferred signal peptide sequence was obtained according to the expression level of the target protein. In this study, the effects of 13 different signal peptides SP1-SP13 on the expression level of the target protein were compared, and *Gaussia luc* was used as a control. The results are shown in FIG. 2. The results indicated that the protein expression level of the SP1 signal peptide was the highest.

**Table 1 Eukaryotic expression signal peptides**

| Signal peptide numbering | Amino acid source and sequence | SEQ ID NO. |
|---|---|---|
| SP1 | Human OSM: MGVLLTQRTLLSLVLALLFPSMASM | 16 |
| SP2 | Human alphaherpesvirus: MGGTAARLGAVILFVVIVGLHGVRG | 17 |
| SP3 | Mouse Ig Kappa: METDTLLLWVLLLWVPGSTGD | 18 |
| SP4 | Human IL-2: MYRMQLLSCIALSLALVTNS | 19 |
| SP5 | Human Chymotrypsinogen: MAFLWLLSCWALLGTTFG | 20 |
| SP6 | Human trypsinogen-2: MNLLLILTFVAAAVA | 21 |
| SP7 | Albumin(HSA): MKWVTFISLLFSSAYS | 22 |
| SP8 | Human VEGF-A: MNFLLSWVHWSLALLLYLHHAKWSQA | 23 |
| SP9 | Human Fibulin-1: MERAAPSRRVPLPLLLLGGLALLAAGVDA | 24 |
| SP10 | Human Vitronectin: MAPLRPLLILALLAWVALA | 25 |
| SP11 | Human Complement factor H: MRLLAKIICLMLWAICVA | 26 |
| SP12 | Human Opticin: MRLLAFLSLLALVLQETGT | 27 |
| SP13 | Human VEGFR-1: MVSYWDTGVLLCALLSCLLLTGSSSG | 28 |
| Control group | *Gaussia luc*: MGVKVLFALICIAVAEA | *29* |

### Example 3 Screening of Fc segment mutants of target proteins

In the experiment, based on Aflibercept (SEQ ID NO. 14), multiple groups of Fc single-point and multi-point mutants were designed, and the coding genes of each mutant fragment Variant1-12 were obtained by the PCR method after designing site-directed mutagenesis primers. The PCR product was purified and digested with endonuclease DpnI for 30 minutes. After the digestion product was transformed into *Escherichia coli,* clones were selected and sequenced to verify the sequences.

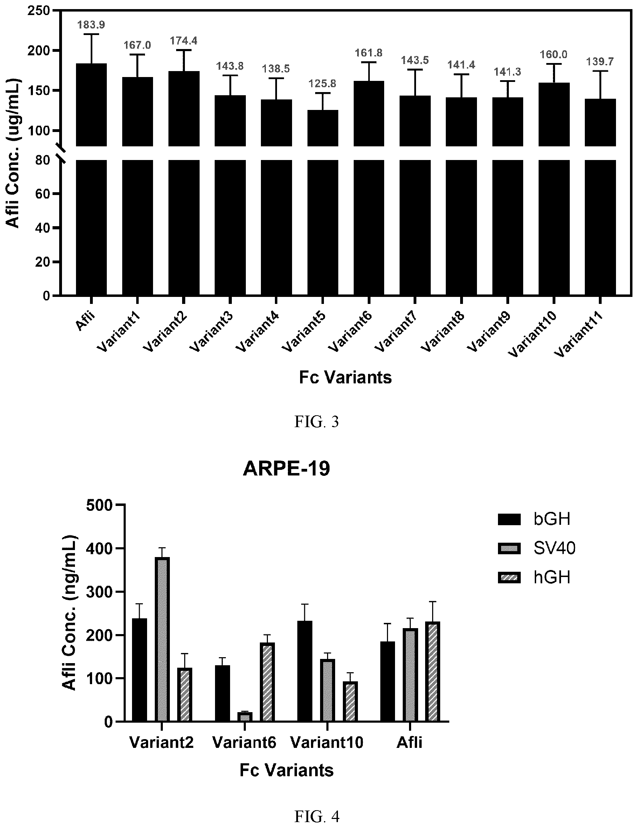

After the clones with correct sequencing were expanded and cultured, a large number of plasmids were extracted with endotoxin removal. Then the Expi293F cells were transiently transfected by the PEI method. The cells were cultured in CD05 medium containing glutamine for 5 days, and then the cell supernatant was taken and the expression level of Aflibercept was detected by ELISA. Meanwhile, the target protein in the cell culture supernatant was purified using Protein A affinity chromatography. The quantification of the protein was performed by the BCA method. The expression levels in the supernatants of the mutant molecules and their affinities for FcRn were investigated. The results are shown in FIG. 3 and Table 2. After the Aflibercept molecule was modified by Fc mutation, Variant1, 2, 6 and 10 mutants did not bind to FcRn, and there was no significant difference in the protein expression levels compared with those before mutation. Variant12 was a positive mutant in terms of the affinity in the Fc region, which increased its affinity with FcRn.

**Table 2 Results of FcRn affinity determination of Fc mutant proteins**

| ID | Affinity KD (M) |
|---|---|
| Afli | 1.37E-7 |
| Variant1 | ND |
| Variant2 | ND |
| Variant6 | ND |
| Variant10 | ND |
| Variant12 | 3.70E-8 |

| | |
|---|---|
| Note: ND indicates not detected. | |

### Example 4 Screening of polyadenylation sequences (PolyA)

In order to achieve efficient pre-mRNA processing, it is necessary to construct efficient polyadenylation sequences contained behind the transgene to form an appropriate PolyA tail at the 3' end of the RNA. The main choices for PolyA sequences in rAAV vectors are bGH PolyA and SV40 PolyA sequences. Different PolyA sequences may increase transcription levels through their interaction with other expression elements. Therefore, in the experiment, three different PolyA sequences were tested to obtain the optimal gene expression and virus production.

The preferred Fc-mutated Aflibercept mutant molecules and the non-mutated Aflibercept molecule (referred to as "Afli" for short) were selected, and these target gene fragments were inserted into the pAAV-MCS vector (containing hGH PolyA). Meanwhile, the PolyA sequences were replaced with bGH PolyA and SV40 PolyA sequences respectively. The constructed various AAV vectors were transiently transfected into ARPE-19 cells and HEK293 cells using Lipofectamine 2000 transfection reagent, and the expression levels of the target proteins were detected 72 hours later.

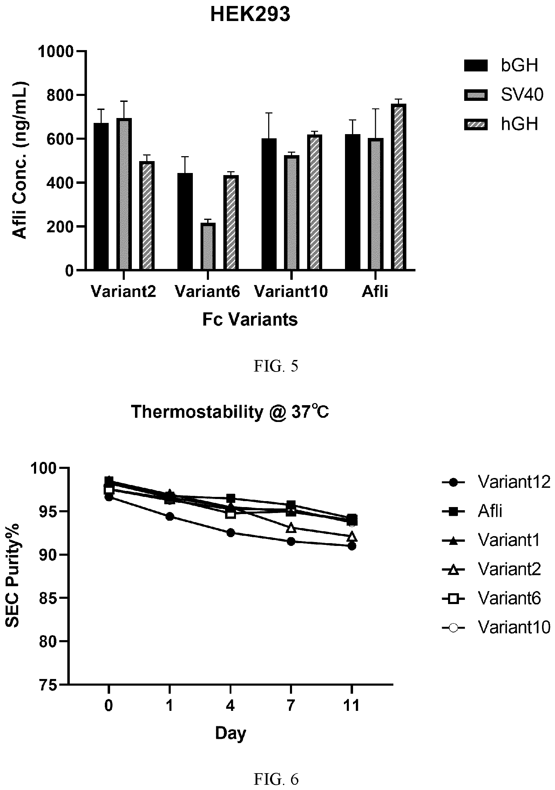

The effects of inserting 3 different PolyA sequences into 4 molecules, i.e. Afli, Variant2, 6 and 10, on the expression levels of the target proteins were tested. The results are shown in FIG. 4 (expression results of the transiently transfected ARPE-19 cells) and FIG. 5 (expression results of the transiently transfected HEK293 cells). The results indicated that PolyA had different effects on the expression efficiencies of different variant molecules, and different molecules preferred different PolyA elements.

### Example 5 Thermostability analysis of fusion proteins

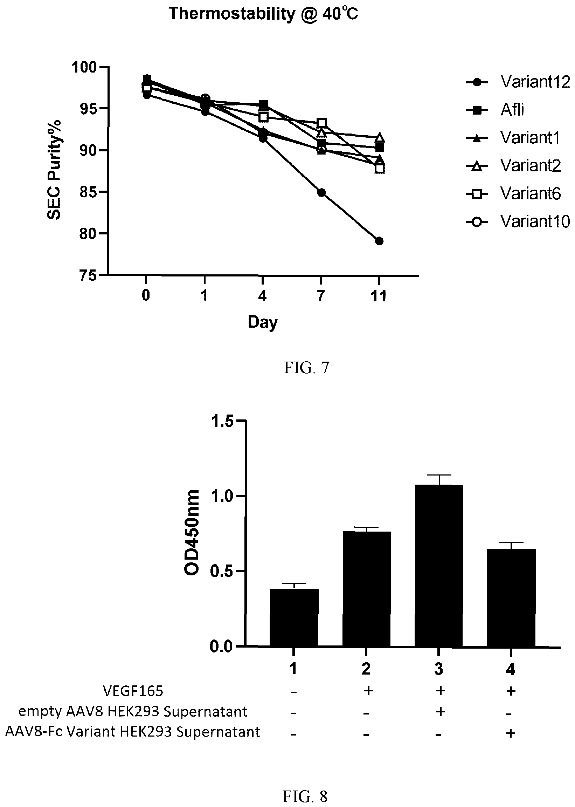

In this study, the thermostability of Aflibercept mutant protein molecules under different heating conditions was tested. The MAbPac SEC-1 from Thermo Fisher was selected as the SEC analysis column. The results are shown in FIG. 6 (results of thermostability analysis of candidate Fc-engineered VEGF receptor recombinant fusion proteins at 37 C) and FIG. 7 (results of thermostability analysis of candidate Fc-engineered VEGF receptor recombinant fusion proteins at 40°C). The results indicated that the SEC purity of each protein molecule showed a decreasing trend after storage at 37°C and 40°C for a certain period of time, and there was no significant difference in thermostability of each protein molecule except Variant12.

### Example 6 Pharmacokinetics of single intravitreal injection of candidate proteins

The PK behaviors of differently engineered Variant2, Variant10 and the control group Aflibercept (hereinafter referred to as Afli, SEQ ID NO. 14) after a single intravitreal injection into both eyes of rabbits were investigated, so as to provide data support for the design and optimization of AAV virus packaging vector genes in the next step. The protein doses were all 1.25 mg/eye, and the administration volumes were 50 µL/eye. After New Zealand rabbits were anesthetized with pentobarbital sodium, the eyes to be injected were disinfected with a povidone-iodine solution, and the candidate proteins were injected into both eyes through the vitreous body at the set doses with a volume of 50 µ/eye. One to two drops of oxybuprocaine hydrochloride eye drops were dropped into the eyes to be injected for surface anesthesia before the injection operation was performed. After the intravitreal injection, about 1-2 drops of ofloxacin eye ointment were added to both eyes of each group of rabbits to keep the cornea moist and prevent infection. From 1 hour to 672 hours after administration, eye tissues were collected at different time points. The retina/choroid plexus, aqueous humor and vitreous body were separated, and the supernatants were obtained after tissue homogenization. Meanwhile, venous blood samples were also collected. The concentrations of protein drugs in the eye tissues and serum were detected by the ELISA method.

For the 3 tissue samples of the vitreous body, aqueous humor and retinal-choroidal plexus, the concentration means of each group of animals were plotted against the time points to draw the drug-time curves, and PK analysis was performed using a one-compartment model. The results are shown in Table 3. Comparing the elimination half-life t1/2 and the area under the drug-time curve AUC₀₋ₜ of each molecule, the half-life t_{1/2} of the Variant10 molecule was 1.03-1.76 times that of Afli, and the exposure amount AUC was 1.19-1.84 times that of Afli; and the half-life t_{1/2} of the Variant2 molecule was 1.15-1.40 times that of Afli, and the exposure amount AUC was 1.02-1.78 times that of Afli. The concentration means of serum samples were plotted against the time points to draw the drug-time curve. A non-compartment model was used to calculate the drug exposure amount AUC of each protein molecule after entering the bloodstream through the eyes (see Table 4). The average AUC of Variant10 was 74.2% lower than that of Afli, and the average AUC of Variant2 was 54.9% lower than that of Afli.

**Table 3 PK analysis parameters of each mutant molecule in ocular tissues (one-compartment model)**

| PK parameters | | t_{1/2} (h) | | AUC₀₋₁ (µg/ml*h) | |
|---|---|---|---|---|---|
| Vitreous body | Afli | 86.255 | | 144551.218 | |
| | Variant2 | 99.405 | | 149234.702 | |
| | Variant10 | 122.948 | | 206656.847 | |
| Aqueous humor | Afli | 106.226 | | 5733.850 | |
| | Variant2 | 122.840 | | 10185.780 | |
| | Variant10 | 109.514 | | 10525.875 | |
| Retinal-choroidal plexus | Afli | 61.858 | | 3872.318 | |
| | Variant2 | 86.356 | | 3946.785 | |
| | Variant10 | 109.132 | | 4610.550 | |

**Table 4 Summary of serum exposure amount AUC of each mutant molecule (non-compartmental model)**

| Test sample | Afli | | Variant2 | | Variant1 0 |
|---|---|---|---|---|---|
| Serum mean AUC₀₋ₜ (ng/ml*h) | 24020.202 | | 10821.94 4 | | 6188.621 |

### Example 7 AAV8-Afli Fc Variant10 AAV viral sample preparation

### 1. Variant 10 vector construction:

(1) The amino acid sequence of Aflibercept was obtained from the DrugBank database (accession number DB08885, SEQ ID NO. 14). Using the codon-optimized synthesized Aflibercept as a template, the Aflibercept fragment was amplified by the PCR method. Meanwhile, the signal peptide derived from *Gaussia luc* (sequence information is shown in Table 1) was added, and this fragment was inserted into the PTT5 vector double-digested with NotI and BamHI. After sequencing verification was correct, the Aflibercept-PTT5 vector was obtained.
(2) Using the Aflibercept-PTT5 vector as a template, site-directed mutagenesis primers for the H310E mutation in the Fc fragment were designed. Then the coding fragment of the Variant10 mutant was obtained by the PCR method. After the PCR products were purified, they were digested with the restriction endonuclease DpnI for 30 minutes. After the digestion product was transformed into *Escherichia coli,* clones were selected and sequenced to verify the sequences. The vector with correct sequencing verification was the Variant10-PTT5 vector.
(3) Using Variant10-PTT5 as a template, the Variant10 coding fragment was amplified by the PCR method and then inserted into the pAAV-MCS plasmid double-digested with EcoRI and HindIII by the homologous recombination method. After transformation into *Escherichia coli,* clones were selected and sequenced to verify the sequences. Meanwhile, the vector with correct sequencing was digested with SmaI to verify whether there was any deletion in the ITR. The verified correct vector was named Afli Fc Variant10.
(4) The AAV vector verified to be correct in step (3) was double-digested with HindIII and RsrII. Meanwhile, the bGHpolyA fragment was obtained by the PCR method (with the template derived from pCMV3-3-Flag). After purification of the above double-digested vector and the PCR-obtained bGHpolyA fragment, they were connected by homologous recombination. After transformation into *Escherichia coli,* clones were selected and sequenced to verify the sequences. Meanwhile, the vector with correct sequencing was digested with SmaI to verify whether there was any deletion in the ITR. After completion of validation, the target plasmid Variant 10-pAAV (as shown in SEQ ID NO. 12) was obtained.

### 2. AAV virus packaging

The AAV virus was packaged by the method of co-transfecting Expi293F cells with three plasmids, where the three plasmids were the helper packaging plasmid, the AAV8 rep-cap plasmid, and the transgenic plasmid.

### (1) Plasmid amplification and extraction

The Variant 10-pAAV vector constructed in Step 1, the AAV8 rep-cap plasmid and the pHelper helper plasmid needed to be extracted in large quantities at a concentration greater than 1 µg/µL, and the A260/280 ratio between 1.8 and 2.0 was used for virus packaging.

### (2) Virus packaging

Expi293F cells were cultured in a serum-free suspension until the cell density reached 1E+6 cells/ml. The extracted three plasmids were mixed at a molar ratio of 1:1:1. Then, the plasmid DNA was mixed with the PEIpro transfection reagent at a mass ratio of 1:2. After incubation at room temperature for 20 minutes, the mixture was slowly added to the cell suspension (with a ratio of 1 mL of cells to 1 µg of the three plasmids) and mixed evenly. The mixture was then placed in a shaker for culture at 37°C with 8% CO₂ for 3 days, and the cell suspension was collected.

### (3) AAV virus concentration

1) The cell suspension was centrifuged at 10,000 g for 10 min. The resulting centrifugation supernatant was transferred to a new centrifuge tube. The cell pellet obtained was resuspended in a small amount of PBS solution, and then the cells were lysed by the freeze-thaw method. After freeze-thawing, the cells were centrifuged again at 10,000 g for 10 min, and the resulting centrifugation supernatant was collected.
2) The supernatants collected from the two centrifugations were mixed together and filtered through a 0.45 µm filter to remove impurities.
3) 1/2 of the volume of 1M NaCl and 10% PEG8000 solution was added, mixed evenly and left at 4°C overnight.
4) The mixture was centrifuged at 12,000 rpm for 2 h. The supernatant was discarded. The virus pellet was dissolved in an appropriate amount of PBS solution. After it was completely dissolved, the solution was filtered through a 0.22 µm filter for sterilization.
5) Benzonase nuclease was added to digest and remove the residual plasmid DNA (with a final concentration of 50 U/ml). The tube cap was closed and the tube was inverted several times to mix thoroughly. Then, it was incubated at 37°C for 30 minutes.
6) The solution was filtered through a 0.45 µm syringe filter and the filtrate was taken as the concentrated AAV virus.

### (4) AAV purification

1) Solid CsCl was added to the virus concentrate until the density was 1.41 g/ml (refractive index was 1.372).
2) The sample was transferred into an ultracentrifuge tube and the remaining space of the centrifuge tube was filled with the pre-prepared 1.41 g/ml CsCl solution.
3) The tube was centrifuged at 175,000 g for 24 hours to form a density gradient. Samples of different densities were collected step by step in sequence, and samples were taken for titer determination. The components enriched with AAV particles were collected.
4) The above process was repeated once.
5) The virus was transferred into a 100 kDa dialysis bag and dialyzed against the dialysis buffer overnight at 4°C to remove salts. The dialysis buffer consisted of PBS containing 0.001% Pluronic F68 with a pH of 7.2. The sample harvested after dialysis was the purified AAV virus AAV8-Afli Fc Variant10, which can be used for in vitro and in vivo analysis and testing.
6) The purified virus was stored in an ultra-low temperature refrigerator at -80°C.

All the AAV viruses used in the present invention were prepared by the above methods. Specific AAV vectors, expression elements, and mutant VEGF receptor recombinant fusion proteins or VEGF recombinant antibodies were selected as needed. After replacing Aflibercept with Bevacizumab and introducing the H310E mutation in the Fc fragment, the plasmid constructed by the same method as above was shown as SEQ ID NO. 13. The AAV virus constructed by using this plasmid with the same method as above was named AAV8-Bevacizumab Fc Variant10.

### Example 8 Biological activity of proteins expressed by AAV vector molecules

The verification of the biological activity of the expression products of candidate vector molecules is an important consideration in the pharmaceutical evaluation of gene therapy. In this study, HEK293T cells were infected with AAV8-Afli Fc Variant 10 and AAV8 empty vector prepared in Example 7 at an MOI of 2E+5, respectively, and the cell supernatant was collected 72 h later. 6E+3 HUVEC cells were seeded in a 96-well plate (50 µL per well) and cultured in a basal medium containing 100 ng/ml VEGF165. After 4-6 h, 50 µL of virus-infected cell supernatant was added, and the culture was continued for 72 h. 10 µL of CCK-8 solution was added to each well, and then the plate was placed at 37°C for incubation. After 3 h, OD 450 nm was read. The results are shown in FIG. 8. It was indicated that the expression products of the candidate vector molecules had an inhibitory effect on the proliferation of HUVEC cells. Specifically, the expression products could bind to VEGF and then inhibit the proliferative effect of VEGF on HUVEC cells.

### Example 9 Transduction expression of AAV vector molecules via different routes of administration

Male C57BL/6J mice aged 8-10 weeks and at the SPF grade were raised in the laboratory for 3-5 days. Before grouping, general ophthalmic examinations, fundus photography (FP), and fundus fluorescein angiography (FFA) were performed on the animals to screen the qualified animals for the experiment. The qualified mice were screened and randomly grouped according to the body weight before administration and divided into 4 groups. Taking the AAV virus AAV8-Afli Fc Variant10 prepared in Example 7 as the test sample, the animal grouping and administration treatments are shown in Table 7 below:

**Table 7 Table of mouse grouping and administration doses via different routes**

| Group | Test sample/control sample | Administration | | | Number of animals |
|---|---|---|---|---|---|
| | | Route | Dose (GC/eye) | Volume (µL/eye) | |
| 1 | AAV8-Afli Fc Variant10 | Intravitreal injection | 1.00E+11 | 1 | 4 |
| 2 | AAV8-Afli Fc Variant10 | Suprachoroidal injection | 1.00E+11 | 1 | 4 |
| 3 | AAV8-Afli Fc Variant10 | Subretinal injection | 1.00E+10 | 1 | 4 |
| 4 | Vehicle | Subretinal injection | NA | 1 | 4 |

| | | | | | |
|---|---|---|---|---|---|
| Note: the vehicles used in the examples of the present invention are all PBS containing 0.001% (W/V) Pluronic F68 with a pH of 7.2. | | | | | |

The test sample was diluted with the vehicle if necessary according to the dose requirements during use.

Each mouse was administered a single dose to both eyes. Administration methods were as follows:
□ Animals needed to be anesthetized before administration. The amount of anesthetic was calculated based on the animal's body weight measured most recently. Heat preservation measures (e.g. covered with blankets) were taken from anesthesia to recovery.
□ The injection needle and the syringe were connected (different injection needles and syringes were used for each group of animals). The required amount of the test sample/control sample solution was drawn for administration, and the administration was performed under an operating microscope.

Subretinal injection: a coverslip was placed on the cornea, and air between them was removed with carbomer eye drops. A disposable insulin needle was used to perform sclera and choroid puncture at a small angle to form a channel. The insulin needle was withdrawn, and a microsyringe was replaced to enter through the channel opening until it reached the subretinal space for injection.

Intravitreal injection: a coverslip was placed on the cornea, and air between them was removed with carbomer eye drops. A disposable insulin needle was used to perform sclera and choroid puncture at a small angle to form a channel. The insulin needle was withdrawn, and a microsyringe was replaced to enter through the channel opening into the vitreous cavity for injection.

Suprachoroidal injection: a disposable insulin needle was used to perform sclera puncture to form a channel. The insulin needle was withdrawn, and a microsyringe was replaced to enter through the channel opening into the suprachoroidal space for injection.

After the administration was completed, levofloxacin eye drops and/or ofloxacin eye ointment were administered 2-3 times a day for 3 consecutive days

On the 8th day after administration, the bilateral eyeballs of the animals were collected. 200 µL of PBS containing protease inhibitors was added to each eye. Homogenization was performed using a tissue homogenizer. After centrifugation (5000 g, 5min, 4°C), the supernatant was taken and temporarily stored at -60°C or below.

The deep-well ELISA plate was coated with VEGF165 protein. After washing, HRP-conjugated goat anti-human IgG (Fc-specific) antibody was added to detect Aflibercept captured on the plate. After the final washing step, TMB substrate solution was added, and the HRP catalyzed the substrate to undergo a color reaction. Finally, a stop solution was added to terminate the reaction. The solution changed from blue to yellow, and the shade of the color was positively correlated with the concentration of Aflibercept protein in the sample. The solution after color reaction was detected with a microplate reader, and the amount of Aflibercept protein in the supernatant of the eyeball tissue homogenate was calculated.

The detection results showed that all the mice had strong Aflibercept protein expression after 3 different administrations.

| Group | Test sample/control sample | Administration | | Expression level of Afli protein (ng/eye±SD) |
|---|---|---|---|---|
| | | Route | Dose (GC/eye) | |
| 1 | AAV8-Afli Fc Variant10 | Intravitreal injection | 1.00E+11 | 309.75±147.36 |
| 2 | AAV8-Afli Fc Variant10 | Suprachoroidal injection | 1.00E+11 | 363.37±165.06 |
| 3 | AAV8-Afli Fc Variant10 | Subretinal injection | 1.00E+10 | 549.65±186.35 |
| 4 | Vehicle | Subretinal injection | NA | ND |

### Example 10 Long-term expression test after AAV vctor molecule injection in mice

Male C57BL/6J mice aged 8-10 weeks and at the SPF grade were raised in the laboratory for 3-5 days. Before grouping, general ophthalmic examinations, fundus photography (FP), and fundus fluorescein angiography (FFA) were performed on the animals to screen the qualified animals for the experiment. Twenty-eight qualified mice were screened and randomly grouped according to the body weight before administration and divided into 7 groups. Taking the AAV virus AAV8-Afli Fc Variant10 prepared in Example 7 as the test sample, the animal grouping and administration treatments are shown in Table 8 below:

**Table 8 Table II of mouse grouping and administration doses**

| Group | Test sample name/batch number | Administration dose (vg/eye) | Administration volume (µl/eye) | Number of animals | Sampling time point (number of weeks after administration) |
|---|---|---|---|---|---|
| 1 | Vehicle control | NA | 1 | 4 | 1 |
| 2 | AAV8- Afli Fc Variant10 | 3E+8 | 1 | 4 | 1 |
| 3 | AAV8- Afli Fc Variant10 | 3E+8 | 1 | 4 | 2 |
| 4 | AAV8- Afli Fc Variant10 | 3E+8 | 1 | 4 | 4 |
| 5 | AAV8- Afli Fc Variant10 | 3E+8 | 1 | 4 | 6 |
| 6 | AAV8- Afli Fc Variant10 | 3E+8 | 1 | 4 | 8 |
| 7 | AAV8- Afli Fc Variant10 | 3E+8 | 1 | 4 | 12 |

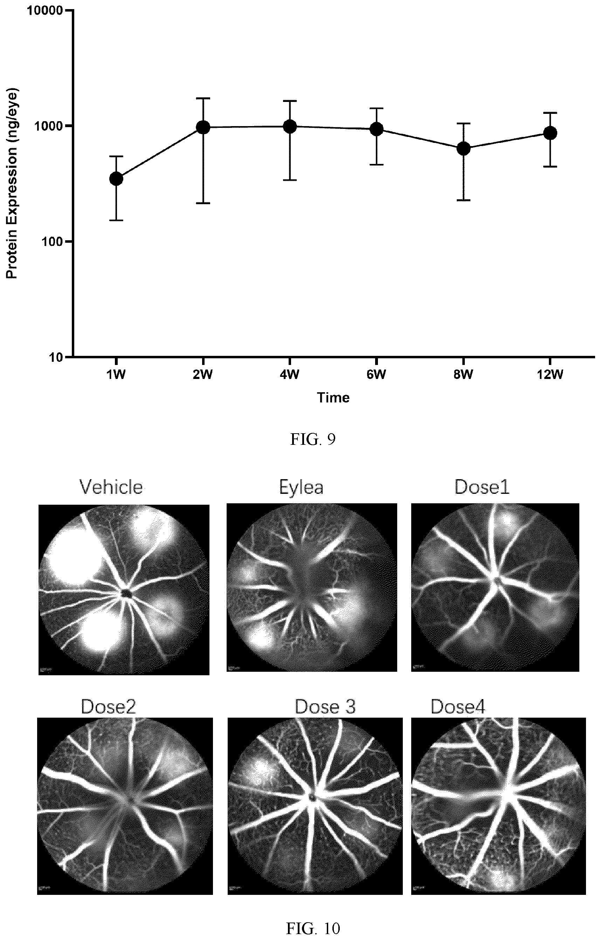

Each mouse was administered a single dose to both eyes. The same subretinal injection administration method and post-treatment method as in Example 9 were adopted, and the expression level of Aflibercept protein in the eyeballs was detected at different time points after administration. The detection results of the amount of Aflibercept protein are shown in Table 9, and the time-expression curve is shown in FIG. 9.

**Table 9 Detection results of protein expression levels at different time points after AAV injection in mice**

| Sampling time point (after administration) | 1w | 2w | 4w | 6w | 8w | 12w |
|---|---|---|---|---|---|---|
| Average protein expression level in eyeballs (ng/eye) ± SD | 349.75± 197.36 | 1102.85± 714.25 | 991.10± 650.97 | 940.40± 478.89 | 638.98± 411.71 | 869.75± 425.12 |

From the test results, it can be seen that the candidate vector molecules had a continuous high expression for up to 12 weeks at a dose of 3E+8 vg/eye. Compared with literature reports, the protein drug Eylea could only be maintained for 4-8 weeks after administration.

### Example 11 Dose escalation test of AAV vector molecules in mice

Male C57BL/6J mice aged 8-10 weeks and at the SPF grade were purchased and raised in the laboratory for 3-5 days. Before grouping, general ophthalmic examinations, fundus photography (FP), OCT and fundus fluorescein angiography (FFA) were performed on the animals to screen the qualified animals for the experiment. Thirty qualified mice were screened and randomly grouped according to the body weight before administration and divided into 6 groups. Taking the AAV virus AAV8-Afli Fc Variant10 prepared in Example 7 as the test sample, the animal grouping and administration treatments are shown in Table 10 (the administration volume of each group was 1 µL/eye):

**Table 10 Detection results of protein expression levels in mice after injection of different doses of AAV**

| Group | Test sample/control sample | Administrati on dose (GC/eye) | Number of mice | Afli expression level (ng/eye) ± SD (7 days) |
|---|---|---|---|---|
| 1 | Vehicle | - | 5 | ND |
| 2 | AAV8- Afli Fc Variant10 | 1E+7 | 5 | 93.6±51.2 |
| 3 | AAV8- Afli Fc Variant10 | 3E+7 | 5 | 158.65±105.5 |
| 4 | AAV8- Afli Fc Variant10 | 1E+8 | 5 | 624.5±349.5 |
| 5 | AAV8- Afli Fc Variant10 | 3E+8 | 5 | 965.5±374.9 |
| 6 | AAV8- Afli Fc Variant10 | 1E+9 | 5 | 1047±420.1 |

Each mouse was administered a single dose to both eyes. The vehicle and different doses of the test sample AAV8-Afli Fc Variant10 were injected into the subretinal space respectively. The administration method was the same as that in Example 9.

General ophthalmic examination, fundus photography (FP), OCT and FFA showed no obvious ocular adverse reactions. From the results of protein expression at different doses, the protein expression showed an obvious dose-dependent correlation.

### Example 12 Candidate carrier molecules for laser-induced mouse CNV models-efficacy test

Male C57BL/6J mice aged 8-10 weeks and at the SPF grade were purchased and raised in the laboratory for 3-5 days. Before grouping, general ophthalmic examinations, fundus photography (FP), and fundus fluorescein angiography (FFA) were performed on the animals to screen the qualified animals for the experiment. Thirty qualified mice were screened and randomly divided into 6 groups according to the body weight before administration. Taking the AAV virus AAV8-Afli Fc Variant10 prepared in Example 7 and the protein drug Eylea^{®} each as the test samples, the animal grouping and administration treatments are shown in Table 11 (the administration volume of each group was 1 µL/eye):

**Table 11 Table III of mouse grouping and administration doses**

| Gro up | Test sample/control sample | Administration | | Number of animals | Admi nistrat ion time |
|---|---|---|---|---|---|
| | | Route | Dose (GC/eye) | | |
| 1 | Vehicle control | Subretinal injection | - | 5 | D1 |
| 2 | Eylea^{®} protein control | Intravitreal injection | 80 µg | 5 | D20 |
| 3 | AAV8- Afli Fc Variant10 | Subretinal injection | 1E+7 | 5 | D1 |
| 4 | AAV8- Afli Fc Variant10 | Subretinal injection | 3E+7 | 5 | D1 |
| 5 | AAV8- Afli Fc Variant10 | Subretinal injection | 1E+8 | 5 | D1 |
| 6 | AAV8- Afli Fc Variant10 | Subretinal injection | 3E+8 | 5 | D1 |

| | | | | | |
|---|---|---|---|---|---|
| Note: 1) the start of administration is counted as D1. 2) Eylea^{®} protein drug can take effect soon after administration, so it is administered after modeling; AAV8-Afli Fc Variant10 reaches a stable expression level about 1-2 weeks after administration and can maintain high expression for a long period of more than 12 weeks, so it is administered before modeling. 3) Eylea^{®} protein drug is the standard treatment method for AMD, and its route of administration is intravitreal injection. | | | | | |

Each mouse was administered a single dose to both eyes. The vehicle and different doses of the test sample AAV8-Afli Fc Variant10 were injected into the subretinal space respectively. The administration method was the same as that in Example 9. Group 2 was the protein control group, with an intravitreal injection dose of 80 µg/eye.

On D17, a laser-induced choroidal neovascularization model was established in both eyes. The planned number of laser burns for each eye was 4. The specific steps were as follows:
1) mydriasis: 1-2 drops of 0.5% compound tropicamide eye drops were dropped into each eye of the animals for mydriasis;
2) anesthesia: the animals were anesthetized by intramuscular injection of Zoletil 50 at 50 mg/kg, 50 mg/mL.
3) laser photocoagulation: carbomer eye drops were dropped into the eyes of the animals before photocoagulation. The fundus laser mirror was placed. After clearly seeing the fundus, laser photocoagulation was performed with a laser of 532 nm wavelength around the optic disc at a distance of about 1.5-2 PD away from the optic disc while avoiding blood vessels (the diameter of the light spot was about 50 µm).
4) animal care: after laser photocoagulation, ofloxacin eye ointment was applied to both eyes of the animals. The animals were placed on a thermal insulation blanket to maintain body temperature before waking up and then put back into the cages after waking up.

On D31, FFA (fundus fluorescein angiography) examination was performed. The typical map is shown in FIG. 10. Before the FFA examination, the animals were given fluorescein sodium injection (10%, 0.02 mL/animal) intravenously.

Analysis indicators: the images of the early stage (within 1.5 min) and the late stage (more than 3 min) of fundus fluorescein angiography were compared. Whether there was choroidal neovascularization and leakage was judged according to whether there was fluorescence leakage in the fundus of the animals, and the degree of fluorescence leakage was graded. The ratio of leakage light spots at all levels and the average score of light spot leakage were calculated.

The grading standards for fluorescence leakage of light spots are as follows: Grade 0 (no fluorescence leakage), Grade 1 (mild fluorescence leakage, leakage area is 1% -50% of a laser spot size), Grade 2 (moderate fluorescence leakage, leakage area is 50%-100% of a laser spot size), Grade 3 (severe fluorescence leakage, leakage area is larger than a laser spot size).

Ratio of light spots at each level (%) = total number of light spots at the corresponding level ÷ total number of 4 types of light spots (i.e., the number of effective light spots) × 100%.

Average score of light spot leakage = [(number of Grade 0 light spots × 0) + (number of Grade 1 light spots × 1) + (number of Grade 2 light spots × 2) + (number of Grade 3 light spots × 3)] ÷ total number of 4 types of light spots (i.e. number of effective light spots), where an effective light spot refers to a light spot that has no severe pre-retinal or sub-retinal hemorrhage nearby and can be completely displayed in the fluorescein angiography.

On D31, the statistical results of the ratio of Grade 3 leakage light spots in the vehicle control group, the Eylea^{®} protein control group, and the AAV8-Afli Fc Variant10 groups with doses of 1E+7, 3E+7, 1E+8, and 3E+8 GC/eye (Table 12) showed that both the Eylea^{®} group and each dose group of AAV8-Afli Fc Variant were significantly lower than the vehicle control group (p ≤ 0.05), and each dose group of AAV8-Fc variant was significantly lower than the Eylea^{®} protein control group (p ≤ 0.05).

**Table 12 Ratio of leakage spots generated by laser-induced CNV models after different doses of AAV injection in mice**

| Test sample/control sample | Administration dose | Ratio of Grade 3 leakage light spots ± SD% |
|---|---|---|
| Vehicle control | NA | 60.4±27.1% |
| Eylea^{®} protein control | 80 µg/eye | 18.8±28.5% |
| AAV8- Afli Fc Variant10 | 1E+7 vg/eye (Does1) | 2.5±7.9% |
| AAV8- Afli Fc Variant10 | 3E+7 vg/eye (Does2) | 0.0±0.0% |
| AAV8- Afli Fc Variant10 | 1E+8 vg/eye (Does3) | 1.8±6.7% |
| AAV8- Afli Fc Variant10 | 3E+8 vg/eye (Does4) | 3.6±9.1% |

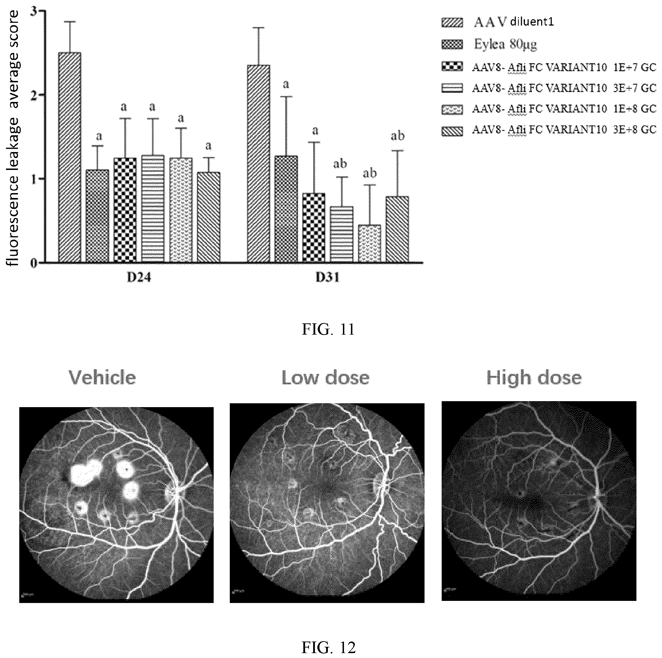

The average score of light spot leakage is shown in FIG. 11. On D31, the average scores of the AAV8-Afli Fc Variant10 groups were all lower than those of the vehicle control group and the Eylea^{®} protein control group. Moreover, compared with D24, the average scores of light spot leakage in each dose group of AAV8-Afli Fc Variant10 were all decreased, while the average score of light spot leakage in the Eylea^{®} protein control group was increased, indicating that each dose group of AAV8-Afli Fc Variant10 could maintain a continuous and stable expression and had more persistent efficacy compared with the Eylea^{®} protein drug.

### Example 13 Efficacy evaluation of AAV vector molecules in non-human primate (cynomolgus monkey) laser model Animals

The ocular structures of non-human primates are similar to those of humans. Cynomolgus monkeys are commonly used non-rodent animal models in non-clinical studies of biological drugs. The laser photocoagulation method was adopted to establish a choroidal neovascularization (CNV) model in cynomolgus monkeys to investigate the inhibitory effect of a single subretinal injection of the AAV virus AAV8-Afli Fc Variant10 prepared in Example 7 on CNV in cynomolgus monkeys.

Nine cynomolgus monkeys that had undergone routine ophthalmic examinations and were confirmed to have no eye diseases were enrolled in the experiment. The AAV virus AAV8-Afli Fc Variant10 prepared in Example 7 was used as the test sample. The grouping and administration are shown in Table 13 as follows:

**Table 13 Grouping and administration doses of cynomolgus monkeys**

| Groupi ng | Induction | Treatment | Numb er of anima ls | Dose | | regime |
|---|---|---|---|---|---|---|
| | | | | GC/eye | µL/eye | |
| 1 | laser | Vehicle control | 3 | -- | 50 | Laser modeling was performed 8 weeks after administration. |
| 2 | laser | Low-dose group | 3 | 1E+9 GC/eye | 50 | |
| 3 | laser | High-dose group | 3 | 1E+10 GC/eye | 50 | |

On D0, the drug was administered at a single dose into the subretinal space. The ocular surface was disinfected with iodophor. Under a special ophthalmic operating microscope, the sclera of the cynomolgus monkey was punctured inside the limbus with a 30G disposable injection needle. Then, a microinjector with a 35G flat needle was inserted along the puncture site, bypassed the lens and reached the vitreous body. After avoiding the main blood vessels, the needle was gradually advanced into the subretinal space, and the drug was slowly injected at a point above the fovea. Immediately after the syringe was withdrawn, the puncture site was compressed with a cotton swab soaked with iodophor for 5 seconds. Each eye was administered with 50 µL of the drug, and both eyes were administered at a single dose.

On D56, the animals were anesthetized with Zoletil (5-10 mg/kg, i.m.). A 532 nm laser was used to burn 8 to 9 laser spots (with the diameter of the light spot being about 50 µm) around the macula. Immediately after the laser modeling, IR photos were taken and OCT scans were performed to confirm whether the modeling was successful. Four weeks after laser modeling, the animals were anesthetized with Zoletil (5-10 mg/kg, i.m.). Then IR photos were taken and SD-OCT scans were performed to examine the healing degree of the laser spots. Early and late fundus fluorescein angiography (FFA) images were taken for leakage scoring. The details of the scoring are shown in Table 14.

**Table 14 Grading standard table of leakage spots**

| Grading of leakage spots | Grading standard |
|---|---|
| Grade I | No strong fluorescence. |
| Grade II | The lesion shows hyperfluorescence in the early or middle stage without leakage. |
| Grade III | The lesion shows hyperfluorescence in the early stage and has leakage in the late stage. |
| Grade IV | The lesion shows bright hyperfluorescence in the early stage, and has leakage in the late stage which extends beyond the boundary of the burned area. |

| | |
|---|---|
| Note: Grade III-IV light spots are leakage lesions. | |

After administration, the content of the target protein in the aqueous humor was continuously detected. The target protein was continuously and stably expressed for 12 weeks and was still under continuous monitoring (while the Eylea^{®} protein drug could only be maintained for 4-8 weeks).

The typical maps of fundus fluorescein angiography (FFA) 4 weeks after laser modeling are shown in FIG. 12. Compared with the vehicle group, the number of leakage spots in the low-dose group (1E+9 GC/eye) and the high-dose group (1E+10 GC/eye) of the AAV virus AAV8-Afli Fc Variant10 was significantly reduced after laser modeling, with significant differences.

After calculating the light spot area and grading the light spot, a Two-way Anova/Dunnett statistical evaluation was conducted. Both the low-dose group and the high-dose group had significant differences compared with the control group. The statistical results are shown in Table 15.

**Table 15 Statistics of leakage spots after laser-induced CNV modeling in cynomolgus monkeys**

| Gro up | Inductio n | Treatmen t | GC/ey e | Anim al numb er | Eye side | Proportion of Grade III + IV leakage light spots | Signific ance analysis |
|---|---|---|---|---|---|---|---|
| 1 | laser | Vehicle control | -- | P1 | OD | 100.0% | NA |
| | | | | | OS | 100.0% | |
| | | | | P2 | OD | 87.5% | |
| | | | | | OS | 100.0% | |
| | | | | P3 | OD | 87.5% | |
| | | | | | OS | 100.0% | |
| 2 | laser | Low-dose group | 1E+9 GC/ey e | P4 | OD | 0.0% | p<0.001 |
| | | | | | OS | 0.0% | |
| | | | | P5 | OD | 0.0% | |
| | | | | | OS | 0.0% | |
| | | | | P6 | OD | 0.0% | |
| | | | | | OS | 0.0% | |
| 3 | laser | High-dose group | 1E+10 GC/ey e | P7 | OD | 0.0% | p<0.001 |
| | | | | | OS | 0.0% | |
| | | | | P8 | OD | 0.0% | |
| | | | | | OS | 0.0% | |
| | | | | P9 | OD | 0.0% | |
| | | | | | OS | 0.0% | |

| | | | | | | | |
|---|---|---|---|---|---|---|---|
| Note: the significance analysis refers to the significance analysis comparing the experimental group with the vehicle control group. P < 0.05 indicates a statistical difference, P < 0.01 indicates a significant statistical difference, and P < 0.001 indicates an extremely significant statistical difference. | | | | | | | |

### Example 14 Efficacy evaluation of AAV vector molecules in non-human primate (cynomolgus monkey) DL-AAA model animals

One challenge of retinal vascular disease models in species that are very similar to human eyes (such as non-human primates (NHP)) is the lack of chronic vascular leakage and/or the characteristics of neovascular responses in human eAMD, DME and PDR. In the laser-induced CNV model of eAMD in NHP, the transient vascular leakage and neovascular response of the choroidal vasculature persist for 6-8 weeks after the laser disruption of the Bruch's membrane, and then spontaneously regress. Although anti-VEGF treatment accelerates the regression of this induced pathology, in humans, the pathology of CNV resumes and persists after the anti-VEGF drug is cleared. Therefore, establishing a model of persistent and recurrent vascular leakage and neovascularization will greatly facilitate and accelerate the evaluation of long-acting interventions to address the multiple clinical manifestations of pathological vascular instability and neovascularization. The DL-α-aminoadipic acid (DL-AAA) model is a chronic leakage model that has been described in rats and rabbits, and routine candidate drug screening has been performed in these species. DL-AAA is a selective glial cell toxin that has been reported to inhibit the action of glutamine synthetase and impair the broader retinal homeostasis function of Müller cells, resulting in glial dysfunction and death, thus leading to blood-retinal barrier breakdown. The retinal vasculature, retinal segmentation and basal layer boundaries, proportional abundance of retinal neurons and glial cell subtypes, as well as the presence of the macula are homologous in monkeys and humans. A novel NHP model of chronic vascular leakage caused by DL-AAA and a preclinical model of chronic retinal vascular leakage and neovascularization allow for the efficacy screening of short-acting and long-acting anti-angiogenic compounds at multiple stages of disease development.

One cynomolgus monkey (animal number P1) was selected and 30 µL of 50 mmol/L DL-α-AAA (Sigma-Aldrich) was injected subretinally into each of its two eyes. Fundus color photography, fundus fluorescein angiography, macular autofluorescence, optical coherence tomography (OCT), and multifocal electroretinogram examination were performed 1 week before drug injection and 6 weeks and 12 weeks after drug injection in each eye to confirm the successful modeling of persistent retinal neovascularization (PRNV) in the cynomolgus monkey, and the model began to form after 6-8 weeks.

One model animal of persistent retinal neovascularization (PRNV) in cynomolgus monkeys, whose modeling time had exceeded half a year, was selected: 1 animal in the bilateral PRNV model group (animal number P1), the right eye injected with the virus AAV8-Afli Fc Variant10 (1E + 10 GC/eye/50 µL) prepared in Example 7, and the left eye injected with the AAV diluent (vehicle control group, 50 µL/eye). The changes in the long-term ophthalmic FFA examination at Baseline, and 2 weeks, 6 weeks, 8 weeks, 10 weeks and 12 weeks after injection were observed. The currently observed results are shown as follows:

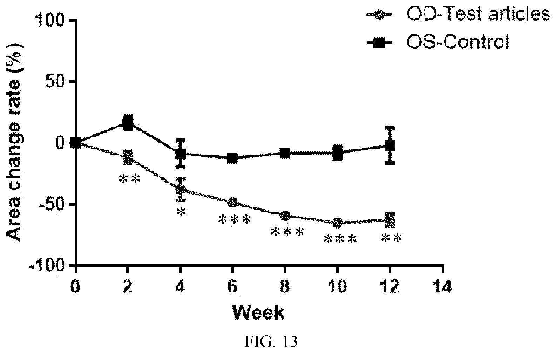

As shown in FIG. 13 (in the figure, *p < 0.05, **p < 0.01, ***p < 0.001 indicate significant statistical differences), the fluorescence leakage in the PRNV modeling and administration group (right eye) had significantly decreased compared with the Baseline at week 2, and continued to decrease every 2 weeks until week 12, with a significant continuous decreasing effect. The fluorescence leakage in the vehicle group of the PRNV modeling monkey (left eye) had no significant changes from week 2 to week 12 compared with the Baseline.

The continuous remission time of the AAV8-Afli Fc Variant10 test sample exceeded the remission time of CNV laser modeling, reflecting the advantage of this model in observing the long-term efficacy of AAV. Statistical analysis found that there were significant differences between the test sample and the vehicle group in both the leakage area and the leakage intensity, indicating that the AAV8-Afli Fc Variant10 adeno-associated virus had significant efficacy in the PRNV model non-human primate animals.

The above-described examples merely express several implementations of the present invention, and the description thereof is relatively specific and detailed, but should not be understood as limiting the scope of the present patent. It should be pointed out that for those of ordinary skill in the art, on the premise of not departing from the concept of the present patent, the above implementations can also be subject to several modifications, combinations and improvements, and all of these belong to the scope of protection of the present patent. Therefore, the scope of protection of the present patent should.

## Claims

1. An Fc fragment mutant of a human IgG1, wherein the mutant has at least one of the following mutations:
T250A, L251A, M252L, 1253A/D/P, S254A, T256A, L309A, H310L/V/A/D/E/Q, Q311A, L314A, M428L/I, H433L/V/A, N434L/V/A, H435L/V/A, Y436A;
mutation numbering is indicated by the EU index.

2. The Fc fragment mutant of human IgG1 according to claim 1, wherein the mutant has one of the following single-point mutations: H310A, H310E or H435A;
or has the following double mutations: H310A/ H435A;
the amino acid sequence is as shown in SEQ ID NO. 1, 2, 3 or 4.

3. A VEGF receptor recombinant fusion protein comprising the Fc fragment mutant of claim 1, wherein the VEGF receptor recombinant fusion protein is formed by fusing a structural domain 2 of VEGFR -1, a structural domain 3 of VEGFR -2, and the Fc fragment mutant of claim 1;
the structural domain 2 of VEGFR -1 is shown in SEQ ID NO. 5;
the structural domain 3 of VEGFR -2 is shown in SEQ ID NO. 6.

4. The VEGF receptor recombinant fusion protein according to claim 3, wherein the amino acid sequence is as shown in SEQ ID NO. 7, 8, 9 or 10.

5. An anti-VEGF recombinant antibody comprising the Fc fragment mutant of claim 1, wherein the Fc fragment is replaced with the Fc fragment mutant of claim 1 on the basis of an anti-VEGF antibody.

6. The anti-VEGF recombinant antibody according to claim 5, wherein the anti-VEGF recombinant antibody is obtained by performing H310 E mutation on a *Bevacizumab* Fc fragment, and has an amino acid sequence as shown in SEQ ID NO. 11.

7. An AAV virus vector expression cassette expressing the VEGF receptor recombinant fusion protein of claim 3 or the anti-VEGF recombinant antibody of claim 5, wherein the expression cassette comprises the following structure of formula I from the 5 ' -3 ' end:
ITR-E1-E2-E3-E4-ITR Formula (I),
wherein:
ITR is a reverse terminal repeat sequence;
E1 is a promoter;
E2 is a signal peptide;
E3 is a nucleotide sequence of the VEGF receptor recombinant fusion protein or the anti-VEGF recombinant antibody;
E4 is a Poly A sequence.

8. The vector expression cassette according to claim 7, wherein the ITR is selected from AAV1, AAV2, AAV3, AAV4, AAV5, AAV6, AAV7, AAV8 or AAV9;
promoter E1 is selected from promoters CMV, CBA, EF 1A, SV 40, PGK1, UBC, CAG, TEF 1, U6, or H1;
signal peptide E2, includes but are not limited to, Human OSM, Hasia Luc, or Albumin (HSA); the Poly A sequence is selected from bGH PolyA, SV40 PolyA, or hGH PolyA.

9. The vector expression cassette according to claim 7, wherein the expression cassette further comprises a regulatory element, and the expression regulatory element comprises but is not limited to a regulatory element with the following functions: (1) an element for regulating and controlling the expression of a target protein; (2) a regulatory element for expressing miRNA and siRNA sequences; (3) an intron; (4) a positioning sequence locating a target protein to a cell nucleus, cytoplasm or various organelles, and being secreted outside the cell; (5) a Kozak sequence; (6) an enhancer; and (7) WPRE.

10. The vector expression cassette according to claim 7, wherein the expression cassette further comprises a tag element, and the tag element comprises, but is not limited to, FLAG, HA, MYC, fluorescent protein, luciferase, SUMO protein, ubiquitin protein, GST, etc.

11. The vector expression cassette according to claim 7, wherein the VEGF receptor recombinant fusion protein or the anti-VEGF recombinant antibody is expressed by the AAV virus vector;
the AAV virus vector includes, but is not limited to, PAAV-CMV, pX 601, pX 551, and PAAV-MCS plasmid.

12. The vector expression cassette according to claim 7, wherein the AAV virus vector expression cassette expressing the VEGF receptor recombinant fusion protein has the sequence shown in SEQ ID NO. 12;
the AAV virus vector expression cassette expressing the anti-VEGF recombinant antibody has the sequence shown in SEQ ID NO. 13.

13. An adeno-associated virus packaging vector system, comprising: an AAV virus vector expression cassette for expressing a VEGF receptor recombinant fusion protein or an anti-VEGF recombinant antibody according to claim 7, a vector carrying AAV rep and cap genes, and an auxiliary virus vector, wherein the vectors are packaged into an AAV virus.

14. The adeno-associated virus packaging vector system according to claim 13, wherein the vector carrying the AAV rep and cap genes includes, but are not limited to: AAV1, AAV2, AAV5, AAV8, AAV9, AAV-R100, AAV-NN, AAV-GL, AAV8-Y447 F, AAV8-Y733 F, AAV8-Y444FY733 F, AAV-DJ or AAV7 M8 vector;
the auxiliary virus vector is a pHelper plasmid.

15. An adeno-associated virus packaging method, wherein the adeno-associated virus packaging vector system of claim 13 is transferred into a host cell for virus packaging;
the host cell is a cell line capable of performing virus replication and stabilizing genetic, including but not limited to cells such as Hela-S3, HEK -293, HEK -293 T, HEK -293 FT, Expi293F, A549 or Sf9.

16. An adeno-associated virus prepared by the adeno-associated virus packaging method of claim 15.

17. The adeno-associated virus according to claim 16, wherein an AAV virus vector expression cassette expressing the VEGF receptor recombinant fusion protein shown in SEQ ID NO. 12, AAV8 Rep-Cap plasmid and pHelper plasmid are transferred into a host cell Expi 293T cell for virus packaging.

18. The adeno-associated virus according to claim 16, wherein an AAV virus vector expression cassette expressing the anti-VEGF recombinant antibody as shown in SEQ ID NO. 13, AAV8 Rep-Cap plasmid and pHelper plasmid are transferred into the host cell Expi 293F for virus packaging.

19. A preparation or formulation or drug comprising the AAV virus vector expression cassette comprising the VEGF receptor recombinant fusion protein or anti-VEGF recombinant antibody of claim 7, or the adeno-associated virus of claim 16.

20. The preparation or formulation or drug according to 19, wherein is a dosage form including, but not limited to, an injection and an ointment.

21. The preparation or formulation or drug according to claim 19, wherein the AAV virus vector expression cassette expressing the VEGF receptor recombinant fusion protein or the anti-VEGF recombinant antibody, or the adeno-associated virus is played as the only active component.

22. A use of the AAV virus vector expression cassette expressing a VEGF receptor recombinant fusion protein or an anti-VEGF recombinant antibody of claim 7, or an application of the adeno-associated virus of claim 16 in the preparation of a preparation or formulation or drug for treating an angiogenesis-related fundus disease.

23. The use according to claim 22, wherein the angiogenesis-related fundus disease comprises age-related macular degeneration, wet macular lesion and diabetic retinopathy.

24. The use according to claim 22, wherein the preparation or formulation or drug is dosed by a single-side eye administration or a double-side eye administration;
dosed by vitreous injection, retinal lower cavity injection, choroidal upper cavity injection, and similar administration modes;
dosed by single dosing or multiple dosing, a total dose of being dosed is 1 × 10⁸-1 × 10¹³ virus genome/eye.
